(19) **Europäisches Patentamt European Patent Office Office européen des brevets**

(11) **EP 4 328 215 B1**

(12) **EUROPEAN PATENT SPECIFICATION**

(45) Date of publication and mention
of the grant of the patent:
**14.01.2026 Bulletin 2026/03**

(21) Application number: **22899139.4**

(22) Date of filing: **29.11.2022**

(51) International Patent Classification (IPC):
$C07C\ 59/01^{(2006.01)}$   $C07C\ 51/48^{(2006.01)}$
$C07C\ 51/43^{(2006.01)}$   $C12P\ 7/52^{(2006.01)}$
$C12N\ 9/88^{(2006.01)}$   $C07C\ 51/02^{(2006.01)}$
$C07C\ 51/41^{(2006.01)}$   $C01B\ 25/32^{(2006.01)}$
$C01F\ 11/46^{(2006.01)}$   $C12P\ 7/42^{(2006.01)}$

(52) Cooperative Patent Classification (CPC):
(C-Sets available)
C07C 51/412; C01B 25/32; C01F 11/46;
C07C 51/02; C07C 51/43; C07C 59/01;
C12N 9/0008; C12N 9/88; C12P 7/42; C12P 7/52;
C12Y 102/01003; C12Y 402/0103; C01P 2002/72;
C01P 2006/80; C01P 2006/82;   (Cont.)

(86) International application number:
**PCT/KR2022/019067**

(87) International publication number:
**WO 2023/096457 (01.06.2023 Gazette 2023/22)**

(54) **METHOD FOR COLLECTING ALKALI METAL SALT HYDRATE AND 3-HYDROXYPROPIONIC ACID**

VERFAHREN ZUR GEWINNUNG VON ALKALIMETALLSALZHYDRAT UND 3-HYDROXYPROPIONSÄURE

PROCÉDÉ DE COLLECTE D'HYDRATE DE SEL DE MÉTAL ALCALIN ET D'ACIDE 3-HYDROXYPROPIONIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.11.2021 KR 20210166974**
**29.11.2021 KR 20210166975**
**29.11.2021 KR 20210167306**
**29.11.2021 KR 20210167409**
**29.11.2021 KR 20210167455**
**29.11.2021 KR 20210167456**
**29.11.2021 KR 20210167457**
**28.11.2022 KR 20220161897**

(43) Date of publication of application:
**28.02.2024 Bulletin 2024/09**

(73) Proprietor: **LG Chem, Ltd.**
**Seoul 07336 (KR)**

(72) Inventors:
• **HAN, Sang Won**
**Daejeon 34122 (KR)**
• **KIM, Taeho**
**Daejeon 34122 (KR)**
• **JEONG, Yongbok**
**Daejeon 34122 (KR)**
• **AHN, Sangbum**
**Daejeon 34122 (KR)**
• **JUNG, Woochul**
**Daejeon 34122 (KR)**
• **KANG, Donggyun**
**Daejeon 34122 (KR)**
• **KIM, Si Min**
**Daejeon 34122 (KR)**
• **KIM, Jimyeong**
**Daejeon 34122 (KR)**
• **BANG, Yongju**
**Daejeon 34122 (KR)**

- **KIM, Jeong Eun**
  **Daejeon 34122 (KR)**
- **CHOE, Jae Hoon**
  **Daejeon 34122 (KR)**

(74) Representative: **Goddar, Heinz J.**
  **Boehmert & Boehmert**
  **Anwaltspartnerschaft mbB**
  **Pettenkoferstrasse 22**
  **80336 München (DE)**

(56) References cited:
  **WO-A1-2015/166098     KR-A- 20210 012 270**
  **US-B2- 8 883 464**

- **J. URBANUS; C.P.M. ROELANDS; D. VERDOES;
  J.H. TER HORST;: "Intensified crystallization in
  complex media: Heuristics for crystallization of
  platform chemicals", CHEMICAL ENGINEERING
  SCIENCE, vol. 77, 13 February 2012 (2012-02-13),
  GB
  , pages 18 - 25, XP028500286, ISSN: 0009-2509,
  DOI: 10.1016/j.ces.2012.02.019**
- **MCDONALD MATTHEW A., SALAMI HOSSEIN,
  HARRIS PATRICK R., LAGERMAN COLTON E.,
  YANG XIAOCHUAN, BOMMARIUS ANDREAS S.,
  GROVER MARTHA A.: "Reactive crystallization:
  a review", REACTION CHEMISTRY &
  ENGINEERING, vol. 6, no. 3, 16 November 2020
  (2020-11-16), pages 364 - 400, XP093068792, DOI:
  10.1039/D0RE00272K**

- **RAMACHANDRAN SUMITRA, PIERRE
  FONTANILLE , ASHOK PANDEY , CHRISTIAN
  LARROCHE: "Gluconic acid: Properties,
  applications and microbial production", FOOD
  TECHNOLOGY AND BIOTECHNOLOGY, vol. 44,
  no. 2, 1 April 2006 (2006-04-01), Croatia
  , pages 185 - 195, XP002670029, ISSN: 1330-9862**

(52) Cooperative Patent Classification (CPC): (Cont.)
  C12N 15/70; C12R 2001/19; Y02A 20/124

  C-Sets
  **C07C 51/02, C07C 59/01;**
  **C07C 51/412, C07C 59/01;**
  **C07C 51/43, C07C 59/01**

**Description**

## FIELD OF THE INVENTION

The present invention relates to a process of recovering alkali metal salt hydrate or alkali earth metal salt hydrate and 3-hydroxypropionic acid.

## BACKGROUND OF THE INVENTION

3-Hydroxypropionic acid (3HP) is a platform compound that can be converted to various chemicals such as acrylic acid, methyl acrylate, and acrylamide. Since being identified as one of the Top 12 value-added bio-chemicals by the US Department of Energy (DOE) in 2004, it has been actively researched in academic and industrial world.

The production of 3-hydroxypropionic acid is mainly carried out by two methods, chemical and biological, but in the case of chemical method, it has been pointed out that the initial material is expensive and that it is non-environmental because toxic substances are generated during the production process, and thus, environmentally friendly bioprocesses have been attracting attention.

3-Hydroxypropionic acid exhibits high hydrophilicity and has high solubility and reactivity with water, unlike other organic acids produced through fermentation processes. This makes it difficult to apply conventional organic acid separation and purification processes such as precipitation and extraction.

The reactive extraction method is a method of extracting an organic acid using an active diluent such as an amine or an alcohol that is highly reactive with the organic acid, which method allows selective extraction of organic acid, and has a relatively high extraction efficiency. Accordingly, an attempt has been made to apply the reaction extraction method even when separating and purifying 3HP. As an example, a method using trioctylamine (TOA) as the amine has been proposed, but there are problems that the extraction efficiency of 3HP is low and a large amount of organic solvent is required. Further, when using tridecylamine as an amine, the extraction efficiency of 3HP is higher than that of TOA, but there is a problem that an emulsion phenomenon occurs in which an organic phase and an aqueous phase are not separated during extraction.

Therefore, there is a need to develop a process of recovering high-purity 3-hydroxypropionic acid with high efficiency.

KR 20210012270 discloses a method of recovery of 3-hydroxypropionic acid from a concentrate comprising the steps: adding calcium sulfate to a concentrate comprising 3-hydroxypropionic acid to form 3-hydroxypropionic acid salt; adding of sulfuric acid to form calcium sulfate and 3-hydroxypropionic acid; and separating and recovering the materials obtained in the previous **step.**

## SUMMARY OF THE INVENTION

### TECHNICAL PROBLEM

It is an object of the present invention to provide a process of recovering an alkali metal salt hydrate or an alkali earth metal salt hydrate that can be recycled together with high-purity 3-hydroxypropionic acid with high efficiency.

### TECHNICAL SOLUTION

Provided herein is a process of recovering alkali metal salt hydrate or alkali earth metal salt hydrate and 3-hydroxypropionic acid, comprising: forming and separating 3-hydroxypropionic acid salt crystal from a concentrate containing 3-hydroxypropionic acid in the presence of an alkali metal salt or an alkali earth metal salt; forming an aqueous solution containing the 3-hydroxypropionic acid salt crystal; and adding an acid to the aqueous solution containing the 3-hydroxypropionic acid salt crystal to form and separate an alkali metal salt hydrate or the alkali earth metal salt hydrate and 3-hydroxypropionic acid.

Further embodiments are disclosed in the dependent claims.

Now, a process of recovering alkali metal salt hydrate or alkali earth metal salt hydrate and 3-hydroxypropionic acid according to embodiments of the present invention will be described in more detail.

It should be understood that, unless steps included in a preparation method described herein are specified as being sequential or consecutive or otherwise stated, one step and another step included in the preparation method should not be construed as being limited to an order described herein. Therefore, it should be understood that an order of steps included in a preparation method can be changed within the range of understanding of those skilled in the art.

In the present invention, the alkali metal includes both alkali metal and alkali earth metal.

According to one embodiment of the present invention, there can be provided a process of recovering alkali metal salt hydrate or alkali earth metal salt hydrate and 3-hydroxypropionic acid, comprising: forming and separating 3-hydroxypropionic acid salt crystal from a concentrate containing 3-hydroxypropionic acid in the presence of an alkali metal salt or an alkali earth metal salt; forming an aqueous solution containing the 3-hydroxypropionic acid salt crystal; and adding an acid to the aqueous solution containing the 3-hydroxypropionic acid salt crystal to form and separate the alkali metal salt hydrate or the alkali earth metal salt hydrate and 3-hydroxypropionic acid.

The present inventors have found through experiments that when 3-hydroxypropionic acid is concentrated in the presence of an alkali metal salt or an alkali earth metal salt to form 3-hydroxypropionic acid salt crystal, high-purity 3-hydroxypropionic acid can be recovered at a high recovery rate, and that when an acid is added to the aqueous solution containing the 3-hydroxypropionic acid salt crystal, not only high-purity hydroxypropionic acid but also alkali metal salt hydrate or alkali earth metal salt hydrate that can be recycled for various purposes can be efficiently recovered together, and completed the present invention.

The 3-hydroxypropionic acid salt crystal may include 3-hydroxypropionic acid calcium salt, and when adding an acid to such 3-hydroxypropionic salt, a precipitate may be formed. For example, when 3-hydroxypropionic acid salt is $Ca(3HP)_2$ and sulfuric acid is used as an acid, a slurry composition containing the precipitate and 3-hydroxypropionic acid can be produced according to the following Reaction Scheme 1. Additionally, the precipitate can be filtered from such a slurry composition to recover 3-hydroxypropionic acid contained in the filtrate.

[Reaction Scheme 1]  $Ca(3HP)_2 + H_2SO_4 + 2H_2O \rightarrow CaSO_4 \cdot 2H_2O + 2(3HP)$

By the way, precipitates such as $CaSO_4 \cdot 2H_2O$ have the property of well absorbing water, and thus, such high-moisture content precipitates can greatly reduce the fluidity of the slurry composition. This not only makes it difficult to transport the slurry composition, but also makes it difficult to filter precipitates from the slurry composition, which causes the problem that a large amount of 3-hydroxypropionic acid remains in the precipitate, and the recovery rate of 3-hydroxypropionic acid is lowered.

On the other hand, in the recovery process according to one embodiment of the invention, since an acid is added to an aqueous solution containing 3-hydroxypropionic acid salt crystal formed from a concentrate containing 3-hydroxypropionic acid in the presence of an alkali metal salt or an alkali earth metal salt, the precipitate formed therefrom has a large particle size and a remarkably low moisture content, so that the fluidity of a slurry composition including the precipitate may be increased. This makes it easier to filter precipitates from the slurry composition, and thus, 3-hydroxypropionic acid can be recovered at a high recovery rate. In addition, alkali metal salt hydrates or alkali earth metal salt hydrates such as $CaSO_4 \cdot 2H_2O$ formed in the above process can be separated and recovered with high efficiency.

On the other hand, at the time point when an acid is added to the aqueous solution containing the 3-hydroxypropionic acid salt crystal, the temperature of the aqueous solution containing the 3-hydroxypropionic acid salt crystal may be 0°C or more and 90°C or less, more specifically, 0°C or more, 15°C or more, 30°C or more, 35°C or more, 40°C or more, 45°C or more, 50°C or more, or 55°C or more, and may be 90°C or less, 80°C or less, 75°C or less, 70°C or less, or 65°C or less.

The particle size and moisture content of the precipitate may be affected by the temperature conditions of the step of adding the acid. For example, when the temperature of the aqueous solution is 25°C or more in the step of adding the acid, the particle size (based on $D_{50}$) of the precipitate may be 30 μm or more, and when the temperature of the aqueous solution is 60°C or more, the particle size (based on $D_{50}$) of the precipitate may be 120 μm or more. That is, when an acid is added to the 3-hydroxypropionic acid salt crystal under the above-mentioned temperature conditions, the precipitate may have large particle size and low moisture content.

In the step of adding the acid, a precipitate represented by the following structural formula 3 can be formed.

[Structural Formula 3]  $Cation(Anion) \cdot pH_2O$

in the structural formula 3, the Cation is the cation of the alkali metal salt or the alkali earth metal salt, the Anion is the anion of the acid, and p is the number of water molecules in a hydrate, which is an integer of 1 or more.

The cation may be, for example, $Na^+$, $Mg^{2+}$ or $Ca^{2+}$, but when it is $Mg^{2+}$ or $Ca^{2+}$, 3-hydroxypropionic acid salt crystal can be formed more effectively. In addition, the anion may be $SO_4^{2-}$, $PO_4^{3-}$ or $CO_3^{2-}$, but is not limited thereto.

Further, the particle size of the precipitate may be 0.5 μm or more, 1.0 μm or more, or 1.5 μm or more, and may be 500.0 μm or less, 400.0 μm or less, 300.0 μm or less, 200.0 μm or less, 100 μm or less, 80 μm or less, 50 μm or less, 30 μm or less, 20 μm or less, 8.0 μm or less, 7.0 μm or less, 6.0 μm or less, or 5.0 μm or less. The precipitate satisfies the above-mentioned particle size, and thus, the moisture content of the precipitate can be lowered, which can increase the fluidity of a slurry containing the precipitate and facilitate filtration of the precipitates from the slurry. The particle size of the precipitate can be measured by a scanning electron microscopy (SEM).

The precipitate may exhibit various particle shapes such as angular, spherical, plate-shape, and needle-shape.

At this time, the particle size of the precipitate is measured based on the straight-line distance between crystal planes with the longest distance among the straight-line distances between crystal planes contained in the precipitate.

In addition, the moisture content of the precipitate may be 200% or less, 150% or less, 130% or less, 110% or less, 100% or less, 80% or less, or 70% or less, and may be 1% or more, 10% or more, or 30% or more. If the moisture content of the precipitate is too high, the fluidity of the slurry containing the precipitate is lowered, which makes it difficult to proceed with the process, and makes it difficult to separate the precipitate from the slurry, thus making it difficult to recover 3-hydroxypropionic acid in high yield.

On the other hand, the aqueous solution containing the 3-hydroxypropionic acid salt crystal can ensure reaction efficiency due to the addition of the acid only when an aqueous solution of a predetermined content or more contains 3-hydroxypropionic acid salt crystal. In addition, since the relative content can be increased compared to other by-products, the purity and efficiency can be further increased during the recovery of 3-hydroxypropionic acid.

For example, the aqueous solution containing 3-hydroxypropionic acid salt crystal may contain 3-hydroxypropionic acid salt crystal in an amount of 100 g/L or more. More specifically, the aqueous solution containing 3-hydroxypropionic acid salt crystal may contain the 3-hydroxypropionic acid salt crystal in an amount of 100 g/L or more, 150 g/L or more, 200 g/L or more, 300 g/L or more, and 1,000 g/L or less, and 900 g/L or less, 800 g/L or less, 700 g/L or less, 650 g/L or less.

When adding an acid to the aqueous solution containing 3-hydroxypropionic acid salt crystal, the concentration and amount of the acid added can be determined in consideration of the amount of 3-hydroxypropionic acid salt crystal in the aqueous solution.

For example, 0.1 M to 10 M acid can be added in an amount of 10% or 500% of the volume of the aqueous solution containing 3-hydroxypropionic acid salt crystal, or the added acid may be used in an amount of 20 wt.% or more, or 30 wt.% or more, and may be used in an amount of 80 wt.% or less, 70 wt.% or less, 60 wt.% or less, or 50 wt.% or less with respect to 100 wt.% of the 3-hydroxypropionic acid salt crystal.

When the content of the acid added is too small, the conversion rate from 3-hydroxypropionic acid salt crystal to 3-hydroxypropionic acid is low, and the content of 3-hydroxypropionic acid recovered may become low. When the content of the acid added is too large, the concentration of unreacted cations and anions remaining in the finally obtained 3-hydroxypropionic acid aqueous solution becomes too high, which may adversely affect the progress of subsequent processes such as preparation process of polylactic acid (PLA), or preparation process of bio-acrylic acid (Bio-AA).

In the recovery process, the acid may be phosphoric acid, and the alkali metal salt hydrate or the alkali earth metal salt hydrate may be phosphoric acid salt. That is, in the recovery process, phosphoric acid can be added to the aqueous solution containing 3-hydroxypropionic acid salt crystal to thereby form and separate phosphate and 3-hydroxypropionic acid.

The present inventors have found through experiments that when phosphoric acid is added to the aqueous solution containing 3-hydroxypropionic acid salt crystal, not only high-purity hydroxypropionic acid but also phosphoric acid salt that can be recycled for various purposes can be efficiently recovered together, and completed the present invention. The phosphoric acid salt may be an inorganic phosphoric acid salt or an organic phosphoric acid salt, and the organic phosphoric acid salt can be recovered and recycled through an organic solvent.

When phosphoric acid is added to 3-hydroxypropionic acid salt, a precipitate may be formed. For example, when 3-hydroxypropionic acid salt is $Ca(3HP)_2$ and inorganic phosphoric acid is used as the acid, a slurry composition containing the precipitate and 3-hydroxypropionic acid can be prepared according to the following Reaction Scheme 5, but is not limited thereto. Meanwhile, when organic phosphoric acid is used as an acid, a slurry composition containing the precipitate and 3-hydroxypropionic acid can be prepared according to the following Reaction Scheme 6, but is not limited thereto. Further, the precipitate can be filtered from such a slurry composition to recover 3-hydroxypropionic acid contained in the filtrate.

[Reaction Scheme 5]          $Ca(3HP)_2 + H_3POn \rightarrow CaHPOn + 2(3HP)$

in Reaction Scheme 5,
n is an integer of 2 to 5.

[Reaction Scheme 6]          $3Ca(3HP)_2 + 2[RO_z\text{-}H_3PO_m] \rightarrow Ca_3[RO_z\text{-}(PO_m)]_2 + 6(3HP)$

in Reaction Scheme 6,

R may be an alkyl group having 1 to 6 carbon atoms, a cycloalkyl group having 3 to 6 carbon atoms, or a phenyl group, z is 0 or 1, and
m is an integer of 2 to 5.

In addition, the phosphoric acid salt contained in the precipitate formed through Reaction Scheme 5 may be CaHPOn, and the precipitate may include a phosphoric acid salt of the following structural formula 3 or a hydrate of the phosphoric acid salt, but is not limited thereto.

[Structural Formula 3] $Ca_xH_y(POn)_z \cdot mH_2O$

wherein,

x and z are each independently an integer of 1 to 10,
y is an integer of 0 to 10,
n is an integer of 2 to 5, and
m is an integer of 0 to 5.

When adding phosphoric acid to the aqueous solution containing 3-hydroxypropionic acid salt crystal, the concentration and amount of the acid added can be determined in consideration of the amount of 3-hydroxypropionic acid salt crystal in the aqueous solution.

For example, phosphoric acid may be added in an amount of 0.8 to 2.0 times the equivalent of 3-hydroxypropionic acid in an aqueous solution containing 3-hydroxypropionic acid salt crystal, and 0.1M to 10M phosphoric acid may be added in an amount of 10% or 500% relative to the volume of the aqueous solution containing 3-hydroxypropionic acid salt crystal, or phosphoric acid may be used in an amount of 20 wt.% or more, or 30 wt.% or more, and may be used in an amount of 80 wt.% or less, 70 wt.% or less, 60 wt.% or less, or 50 wt.% or less, based on 100 wt.% of the 3-hydroxypropionic acid salt crystal.

When the content of the phosphoric acid added is too small, the conversion rate from 3-hydroxypropionic acid salt crystal to 3-hydroxypropionic acid is low, and the content of 3-hydroxypropionic acid recovered may be small. When the content of the acid added is too large, the concentration of unreacted cations and anions remaining in the finally obtained 3-hydroxypropionic acid aqueous solution becomes too high, which may adversely affect the progress of subsequent processes such as preparation process of acrylic acid (AA), poly3-hydroxypropionic acid (P(3HP)), PLH (poly lactate hydracrylate), or preparation process of bio-acrylic acid (Bio-AA). On the other hand, phosphoric acid can be used as a catalyst for the conversion reaction of acrylic acid (AA), and thus, when a small amount of phosphoric acid remains, the preparation process of acrylic acid using phosphoric acid as a catalyst can be progressed advantageously.

The recovery process according to one embodiment of the invention may further comprise separating 3-hydroxypropionic acid salt crystal from the concentrate and preparing an aqueous solution of the 3-hydroxypropionic acid salt crystal, after the step of forming the 3-hydroxypropionic acid salt crystal.

As mentioned above, as the 3-hydroxypropionic acid salt crystal are produced in the fermentation process, the concentrate in which the fermentation liquid has been concentrated may contain a large amount of impurities such as bacterium strains, carbon sources, and alkali metal salts or alkali earth metal salts in addition to the 3-hydroxypropionic acid salt crystal. However, in the process of recovering 3-hydroxypropionic acid according to one embodiment of the invention, the impurities can be removed by solid-liquid separation and recovery of 3-hydroxypropionic acid salt crystal from the concentrate, and the impurities may not be contained even in a solution prepared by dissolving the separated 3-hydroxypropionic acid crystals in a solvent such as distilled water.

Therefore, when ammonia is added to the concentrate without separating the 3-hydroxypropionic acid crystals from the concentrate, a large amount of impurities are contained in the concentrate, which may make it difficult to finally recover high-concentration and high-purity 3-hydroxypropionic acid.

For example, the 3-hydroxypropionic acid salt crystal can be separated from the concentrate using a filtration flask, or a vacuum pump, and the separated 3-hydroxypropionic acid salt crystal can be dissolved in a solvent such as distilled water to produce an aqueous solution of 3-hydroxypropionic acid salt crystal.

The solution containing 3-hydroxypropionic acid salt crystal may contain 3-hydroxypropionic acid salt crystal at a concentration of 100 g/L or more, 150 g/L or more, or 200 g/L or more, and may contain it at a concentration of 800 g/L or less, 750 g/L or less, or 700 g/L or less. If the concentration of the 3-hydroxypropionic acid salt crystal contained in the solution containing 3-hydroxypropionic acid salt crystal is too low, the concentration of 3-hydroxypropionic acid finally recovered may be lowered, and if the concentration of the 3-hydroxypropionic acid salt crystal is too high, 3-hydroxypropionic acid may be precipitated, or the solid phase concentration after precipitation may be too high, and thus, the recovery rate may be reduced due to a decrease in fluidity.

Then, an acid may be added to the aqueous solution of 3-hydroxypropionic acid salt crystal to form a slurry composition containing a precipitate and 3-hydroxypropionic acid, and before adding the acid, an aqueous solution of 3-hydroxypropionic acid salt crystal is stirred in a specific temperature range, and this temperature condition is kept until the time point when the acid is added, thereby forming a precipitate. The precipitate formed under such particular temperature range condition may exhibit large particle size and low moisture content.

In addition, the recovery process according to one embodiment of the invention may further comprise filtering the precipitate and washing the filtered precipitate, after the step of adding the acid.

The precipitate can be filtered from the slurry composition using a filtration flask, or a vacuum pump, the alkali metal salt hydrate or alkali earth metal salt hydrate can be recovered from the precipitate, and the filtered precipitate may be washed to further recover 3-hydroxypropionic acid contained in the precipitate.

Therefore, the 3-hydroxypropionic acid may be contained in a filtrate in which the precipitate has been filtered. In addition, 3-hydroxypropionic acid may be contained even in a washing liquid in which the precipitate has been washed. However, since the washing liquid may contain other impurities in 3-hydroxypropionic acid, 3-hydroxypropionic acid can be recovered from the filtrate in which the washing liquid has been filtered.

Components such as alkali metal salt hydrate or alkali earth metal salt hydrate contained in the precipitate can be confirmed by known methods such as X-ray fluorescence spectrometry (XRF), and the type and crystal form of the material can be confirmed using an X-ray diffraction analyzer (Bruker AXS D4-Endeavor XRD). In addition, the moisture content of the alkali metal salt hydrate or alkali earth metal salt hydrate can be confirmed with a moisture-measuring device, and impurities other than alkali metal salt hydrates or alkali earth metal salt hydrates can be confirmed by known methods such as nuclear magnetic resonance spectroscopy (NMR), high performance liquid chromatography (HPLC), or X-ray fluorescence spectrometry (XRF).

As mentioned above, an alkali metal salt hydrate or alkali earth metal salt hydrate obtained in the step of recovering 3-hydroxypropionic acid derived from an environmentally friendly bioprocess may have characteristics that mineral-derived by-products such as natural radionuclides (radium (226Ra), thorium (232Th), and potassium (40K) do not exist, or the whiteness is high, unlike by-products obtained in the process of recovering 3-hydroxypropionic acid obtained from previously known chemical fixation.

For example, in the process of recovering 3-hydroxypropionic acid derived from an environmentally friendly bioprocess, it is possible to recover alkali metal salt hydrates or alkali earth metal salt hydrates such as $CaSO_4$ that contain gypsum dihydrate, gypsum hemihydrate, and gypsum anhydrite in an amount of at least 90% of the total content, and particularly are free of natural radionuclide.

In one example, the recovery rate of 3-hydroxypropionic acid in the process of recovering 3-hydroxypropionic acid provided by the present invention may be 40% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, but is not limited thereto. The recovery rate may be calculated based on the weight.

The step of adding an acid to the aqueous solution containing 3-hydroxypropionic acid salt crystal to form and separate an alkali metal salt hydrate or alkali earth metal salt hydrate and 3-hydroxypropionic acid may comprise separating the alkali metal salt hydrate or alkali earth metal salt hydrate from the acid-added solution containing the alkali metal salt hydrate or alkali earth metal salt hydrate and 3-hydroxypropionic acid, and electrodialyzing the acid-added solution from which the alkali metal salt hydrate or alkali earth metal salt hydrate has been separated.

In particular, the recovered 3-hydroxypropionic acid may contain various impurities, and some of the 3-hydroxypropionic acid may remain in salt form, wherein the alkali metal salt hydrate or alkali earth metal salt hydrate is first separated, and the remaining filtrate is electrodialyzed, thereby capable of greatly reducing the content of impurities and greatly increasing the purity of 3-hydroxypropionic acid among the finally recovered components.

The electrodialysis may use a constant voltage method or a constant current method, wherein electricity can be applied to an electrodialysis device so that impurities such as other ions can be purified from the acid-added solution in which the alkali metal salt hydrate or alkali earth metal salt hydrate has been separated, and components present as 3-hydroxypropionic acid salts can be converted to 3-hydroxypropionic acid to recover high-purity 3-hydroxypropionic acid.

Conventionally, an attempt was made to charge the fermented liquid directly to an electrodialysis device to remove impurities, but there was a problem that the cation dissociated from 3-hydroxypropionic acid salt clogged a separation membrane or caused a fouling phenomenon, which caused the problem of reduction in recovery process efficiency.

However, in the recovery process of one embodiment of the invention, after first forming 3-hydroxypropionic acid salt crystal, an acid is added thereto to separate an alkali metal salt hydrate or alkali earth metal salt hydrate and 3-hydroxypropionic acid, and then 3-hydroxypropionic acid is recovered by electrodialysis, which can thus prevent the problem of the above-mentioned fouling phenomenon, and can recover high-purity 3-hydroxypropionic acid with high efficiency.

Although the electrodialysis amount during electrodialysis is not particularly limited, it can be selected, for example, from the range of 5 to 50 Flux, or 10 to 30 Flux.

For the electrodialysis, a conventionally well-known electrodialysis device, electrodialysis method, or electrodialysis system can be used. For example, an electrodialysis system may comprise an electrodialysis tank equipped with two opposing electrodes and a cation-selective membrane and/or anion-selective membrane positioned between the electrodes; a supply tank that supplies reactants to the electrodialysis tank; a desalination tank that recovers desalting products formed while the reactants supplied to the electrodialysis tank pass through the electrodialysis tank; and a concentration tank that recovers cations, anions, or salts formed while the reactants supplied to the electrodialysis tank pass through the electrodialysis tank.

The step of electrodialyzing the acid-added solution from which the alkali metal salt hydrate or alkali earth metal salt hydrate has been separated can be carried out until the time point when the electrical conductivity in a desalination tank for recovering the desalting product formed by electrodialysis of the acid-added solution from which the alkali metal salt hydrate or alkali earth metal salt hydrate has been separated is reduced to 50% or less, 40% or less, 30% or less, 20% or less, 10% or less, or 5% or less of the initial value.

If the electrodialysis time or electrodialysis amount increases, the rate at which some impurities are removed becomes higher, but a phenomenon occurs in which the 3-hydroxypropionic acid to be recovered moves to the concentration tank, and the entire process time can increase significantly.

The initial electrical conductivity of the desalination tank is not particularly limited, but it may be 5 mS/cm or more and 200 mS/cm or less, 10 mS/cm or more and 180 mS/cm or less, 20 mS/cm or more and 150 mS/cm or less, or 30 mS/cm or more and 130 mS/cm or less. If the initial electrical conductivity of the desalination tank is too low, it may be difficult to separate the ionic material to be separated, and if the initial electrical conductivity is too high, desalination requires a long time, and the contamination degree of the separation membrane may become high.

After the step of adding an acid to the aqueous solution containing the 3-hydroxypropionic acid salt crystal to form an alkali metal salt hydrate or alkali earth metal salt hydrate and 3-hydroxypropionic acid, a cation exchange resin column can also be used in the step of recovering the formed 3-hydroxypropionic acid.

Specifically, the step of adding an acid to the aqueous solution containing 3-hydroxypropionic acid salt crystal to form and separate an alkali metal salt hydrate or alkali earth metal salt hydrate and 3-hydroxypropionic acid may comprise contacting the formed solution containing 3-hydroxypropionic acid with a cation exchange resin column; and recovering 3-hydroxypropionic acid from a solution contacted with the cation exchange resin column.

The cation exchange resin column serves to convert 3-hydroxypropionic acid salt present in a solution containing 3-hydroxypropionic acid to 3-hydroxypropionic acid, whereby 3-hydroxypropionic acid having higher purity can be separated and recovered with high efficiency.

Meanwhile, the cation exchange resin may be a polymer containing a functional group capable of ion exchange in its matrix, wherein the ion-exchangeable functional groups introduced into the matrix can be in the acid form capable of exchanging hydrogen cation ($H^+$), such as -COOH, -SOOH, and -POOH.

Further, polystyrene, or acrylic polymer can be used as the matrix of the cation exchange resin, and various matrixes such as homopolymers, co-polymers, block polymers and random copolymers can be used.

The particles of the cation exchange resin may have a spherical or irregular shape, and the particle size distribution range of the cation exchange resin may be 0.3 mm to 1.2 mm.

The exchange efficiency of the cation exchange resin may be 1.2 eq to 2.0 eq, or 1.6 eq to 1.9 eq, taking into account the stability of the cation exchange resin and the degree of ion desorption within the cation exchange resin.

In addition, the cation exchange resin may be used alone or in combination of two or more types, and the exchange efficiency varies depending on the type of the resin used, and thus, the resin can be selected in consideration of the particle size, shape, and exchanger.

Meanwhile, the resin volume of the cation exchange resin column may be 10 BV or more, 11 BV or more, or 12 BV or more. The resin volume (BED VOLUME; BV) means the volume occupied by the filler in the column. If the resin volume content of the cation exchange resin column is too low, exchange efficiency decreases during ion exchange, and if the resin volume content is too high, the ion exchange time increases, which may be uneconomical.

Meanwhile, in the recovery process according to one embodiment of the invention, 3-hydroxypropionic acid salt crystal can be formed from the concentrate containing 3-hydroxypropionic acid in the presence of an alkali metal salt or alkali earth metal salt, before the step of adding an acid.

The concentrate containing 3-hydroxypropionic acid may contain 3-hydroxypropionic acid at a concentration of 300 g/L or more, 350 g/L or more, 400 g/L or more, 450 g/L or more, or 500 g/L or more, and may contain 3-hydroxypropionic acid at a concentration of 900 g/L or less, 850 g/L or less, or 800 g/L or less. Whether or not the crystal of 3-hydroxypropionic acid is formed appears to be affected by the presence of an alkali metal salt or alkali earth metal salt, and the concentration of 3-hydroxypropionic acid in the concentrate.

Further, when the concentration of the crystal of 3-hydroxypropionic acid in the concentrate is higher than the water solubility of the crystal of 3-hydroxypropionic acid, the crystal of 3-hydroxypropionic acid can be more easily produced.

For example, the water solubility of $Ca(3HP)_2$, which is a crystal of 3-hydroxypropionic acid, is 450 g/L at room temperature, and thus, when the concentration of 3-hydroxypropionic acid in the concentrate exceeds 450 g/L, the formation of $Ca(3HP)_2$ crystal can be promoted. Further, the water solubility of $Mg(3HP)_2$, which is a crystal of 3-hydroxypropionic acid, is 250 g/L at room temperature, and thus, when the concentration of 3-hydroxypropionic acid in the concentrate exceeds 250 g/L, the formation of Mg(3HP)$_2$ crystal can be promoted.

The 3-hydroxypropionic salt crystal can be formed from a concentrate satisfying the above-mentioned concentration even while containing the alkali metal salt or alkali earth metal salt. The alkali metal salt or alkali earth metal salt may be selected without limitation within the purpose range for forming 3-hydroxypropionic acid salt crystal. For example, the alkali metal salt or alkali earth metal salt may include one or more cations selected from the group consisting of Na$^+$, Mg$^{2+}$ and Ca$^{2+}$, but when a cation of Mg$^{2+}$ or Ca$^{2+}$ or a salt thereof is used, 3-hydroxypropionic salt crystal can be formed more effectively. For example, the alkali metal salt or alkali earth metal salt may be Ca(OH)$_2$, Mg(OH)$_2$ , or a mixture thereof.

The alkali metal salt or alkali earth metal salt is added and remains in the process of producing the 3-hydroxypropionic acid fermentation liquid, or it can be added in the process of forming 3-hydroxypropionic acid salt crystal in the concentrate containing 300 g/L or more of 3-hydroxypropionic acid. Further, the concentration of the alkali metal salt or alkali earth metal salt may be 10% to 100%, or 30% to 90% of the 3-hydroxypropionic acid concentration, and for example, it can be present in the concentrate at a concentration of 10 to 900 g/L, 50 to 800 g/L, 100 to 700 g/L, or 200 to 600 g/L.

Further, the step of forming 3-hydroxypropionic acid salt crystal from the concentrate containing 3-hydroxypropionic acid may further comprise contacting the concentrate of 3-hydroxypropionic acid with a nonsolvent. When contacting the concentrate with the nonsolvent, the 3-hydroxypropionic acid salt crystal can be more easily formed. When the concentrate of the formed 3-hydroxypropionic acid is brought into contact with a nonsolvent in the presence of the alkali metal salt or alkali earth metal salt, pure 3-hydroxypropionic acid salt crystals can be formed by controlling the crystal formation rate as compared to crystals formed at a high rate due to overconcentration, and thereby, the crystal size can be increased. Such 3-hydroxypropionic acid crystals contain very low amounts of impurities inside the crystals, and have excellent filterability due to uniform crystal formation, so that not only it is easy to purify, but it is also excellent in shape stability and heat stability in the crystal state, thereby being able to efficiently mass-produce 3-hydroxypropionic acid having high purity.

For example, when a nonsolvent is brought into contact with a concentrate of the formed 3-hydroxypropionic acid in the presence of an alkali metal salt or alkali earth metal salt, an alkali metal salt or alkali earth metal salt of 3-hydroxypropionic acid is produced, and as the concentration of the produced alkali metal salt or alkali earth metal salt of 3-hydroxypropionic acid increases, fine crystals are formed, and solid-liquid phase separation occurs. After that, as crystallization proceeds, the alkali metal salt or alkali earth metal salt of 3-hydroxypropionic acid grows into solid crystals (3-hydroxypropionic acid salt crystal), and the resultant 3-hydroxypropionic acid salt crystal contain impurities at a very low content while being separated from liquid impurities such as glycerol and 1,3-propanediol. At this time, the nonsolvent serves to promote crystallization of the alkali metal salt or alkali earth metal salt of 3-hydroxypropionic acid, so that the solid-liquid separation ability is increased and 3-hydroxypropionic acid salt crystal having higher purity can be produced.

The volume ratio between the concentrate of 3-hydroxypropionic acid and the nonsolvent may be determined by considering the concentration of 3-hydroxypropionic acid or the volume of the concentrate and nonsolvent. For example, the concentrate and the nonsolvent may be used in a volume ratio of 1:0.5 to 1:20, or 1:0.5 to 1:10, or 1:0.8 to 1:8, or 1:1 to 1:5.

If the volume of the nonsolvent is too small compared to the concentrate, the concentration at which crystals are formed is high, and the rate of crystal formation is increased, so that pure crystal particles are not formed slowly and irregular crystal particles are rapidly formed. Further, the solid-liquid separation ability is low, which may make it difficult to separate liquid impurities from the alkali metal salt or alkali earth metal salt of 3-hydroxypropionic acid to be purified. In addition, if the volume of the nonsolvent is too large compared to the concentrate, the formed crystals may be melted and dissolved in some cases due to the solubility of the nonsolvent, and the time in the crystal filtration process after crystal formation increases, and the amount of waste liquid increases, which may be uneconomical.

The nonsolvent may include an alcohol-based nonsolvent, a ketone-based nonsolvent, a nitrile-based nonsolvent, or a mixture of two or more thereof, and more specifically, at least one alcohol-based nonsolvent may be used.

The ketone-based nonsolvent may include one or more selected from the group consisting of acetone, methyl ethyl ketone, cyclohexanone, diethyl ketone, acetophenone, methyl isobutyl ketone, methylisoamylketone, isophorone and di-(isobutyl)ketone. The alcohol-based nonsolvent may include one or more selected from the group consisting of methanol, ethanol, allyl alcohol, 1-propanol, 2-propanol, 1-butanol, 2-butanol, isobutanol, benzyl alcohol, cyclohexanol, diacetone alcohol, ethylene glycol monoethyl ether, diethylene glycol monomethyl ether, diethylene glycol monoethyl ether, ethylene glycol monobutyl ether, diethylene glycol monobutyl ether, 2-methoxyethanol and 1-decanol. The nitrile-based nonsolvent may include one or more selected from the group consisting of acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile.

The step of contacting the concentrate with a nonsolvent to form 3-hydroxypropionic acid salt crystal may be carried out at a temperature of 0°C or more, 15°C or more, 30°C or more, 35°C or more, 40°C or more, 45°C or more, 50°C or more, or 55°C or more, and may be carried out at a temperature of 100°C or less, 90°C or less, 80°C or less, 75°C or less, 70°C or less, 65°C or less, or 0°C to 100°C.

At this time, the temperature may be adjusted to the above range by adding a non-solvent at the above-described temperature to the concentrate, or by heating or cooling in a state in which the concentrate and the nonsolvent are mixed.

The 3-hydroxypropionic acid salt crystal may be the form shown in the following structural formula 1 or structural formula 2. That is, the 3-hydroxypropionic acid salt crystal may include 3-hydroxypropionic acid salt in the form shown in the structural formula 1 or structural formula 2.

In the structural formula 1 and structural formula 2, Cation means a cation, 3HP means 3-hydroxypropionic acid that binds to the cation, n is the number of 3HP that binds to the cation and means an integer of 1 or more, and in the structural formula 2, m is the number of water molecules that binds to Cation(3HP)n in the hydrate, which is an integer of 1 or more. The cation may be, for example, $Na^+$, $Mg^{2+}$ or $Ca^{2+}$, but in the case of $Mg^{2+}$ or $Ca^{2+}$, 3-hydroxypropionic acid salt crystal may be formed more effectively.

[Structural Formula 1]     $Cation(3HP)_n$

[Structural Formula 2]     $Cation(3HP)_n \cdot mH_2O$

In addition, the step of forming 3-hydroxypropionic acid salt crystal may further comprise stirring the concentrate.

The stirring step can be carried out at a temperature of 0 to 70 degrees Celsius, 0 to 60 degrees Celsius, 0 to 50 degrees Celsius, 0 to 40 degrees Celsius, 0 to 35 degrees Celsius, 0 to 30 degrees Celsius, 10 to 70 degrees Celsius, 10 to 60 degrees Celsius, 10 to 50 degrees Celsius, 10 to 40 degrees Celsius, 10 to 35 degrees Celsius, 10 to 30 degrees Celsius, 15 to 70 degrees Celsius, 15 to 60 degrees Celsius, 15 to 50 degrees Celsius, 15 to 40 degrees Celsius, 15 to 35 degrees Celsius, 15 to 30 degrees Celsius, 20 to 70 degrees Celsius, 20 to 60 degrees Celsius, 20 to 50 degrees Celsius, 20 to 40 degrees Celsius, 20 to 35 degrees Celsius, or 20 to 30 degrees Celsius (e.g., room temperature), and/or under conditions of 100 to 2000rpm, 100 to 1500rpm, 100 to 1000rpm, 100 to 500rpm, 100 to 400rpm, or 200 to 400rpm (e.g., about 300rpm).

The particle size distribution $D_{50}$ of the 3-hydroxypropionic acid salt crystal may be 20 $\mu$m or more, 25 $\mu$m or more, 30 $\mu$m or more, 35 $\mu$m or more, and 300 $\mu$m or less, 250 $\mu$m or less, 200 $\mu$m or less, 150 $\mu$m, 90 $\mu$m or less, 85 $\mu$m or less, 80 $\mu$m or less, 75 $\mu$m or less.

In addition, the particle size distribution $D_{10}$ of the 3-hydroxypropionic acid salt crystal may be 5 $\mu$m or more and 40 $\mu$m or less, 8 $\mu$m or more and 35 $\mu$m or less, 10 $\mu$m or more and 30 $\mu$m or less, and the particle size distribution $D_{90}$ of the 3-hydroxypropionic acid salt crystal may be 50 $\mu$m or more and 200 $\mu$m or less, 60 $\mu$m or more and 190 $\mu$m or less, 65 $\mu$m or more and 180 $\mu$m or less, 70 $\mu$m or more and 175 $\mu$m or less.

The particle size distributions $D_{50}$, $D_{10}$ and $D_{90}$ mean particle diameters at which cumulative volumes of particles reach 50%, 10% and 90%, respectively, in the particle size distribution curve of the particles, wherein the $D_{50}$, $D_{10}$ and $D_{90}$ can be measured using, for example, a laser diffraction method. The laser diffraction method can generally measure particle sizes ranging from a submicron range to several millimeters, and can obtain results with high reproducibility and high resolvability.

If the particle size distributions $D_{50}$, $D_{10}$ and $D_{90}$ of the 3-hydroxypropionic acid salt crystal is too large, impurities that must be removed during crystallization may be contained in the crystals, which results in a reduction in purification efficiency. If the particle size distributions are too small, the liquid permeability during filtration of the crystals may be lowered.

Meanwhile, the $(D_{90}-D_{10})/D_{50}$ of the 3-hydroxypropionic acid salt crystal may be 1.00 or more and 3.00 or less, 1.20 or more and 2.80 or less, 1.40 or more and 2.60 or less, or 1.60 or more and 2.40 or less.

Further, the 3-hydroxypropionic acid salt crystal may have a volume average particle size of 30 $\mu$m or more and 100 $\mu$m or less, 35 $\mu$m or more and 95 $\mu$m or less, or 40 $\mu$m or more and 90 $\mu$m or less, a number average particle size of 1 $\mu$m or more and 30 $\mu$m or less, 3 $\mu$m or more and 25 $\mu$m or less, or 5 $\mu$m or more and 20 $\mu$m or less, and a volume average particle size of 10 $\mu$m or more and 70 $\mu$m or less, 15 $\mu$m or more and 60 $\mu$m or less, or 20 $\mu$m or more and 55 $\mu$m or less.

If the volume average particle size, number average particle size, and volume average particle size of the 3-hydroxypropionic acid salt crystal are too large, impurities that must be removed during crystallization may be contained in the crystals, which results in a reduction in purification efficiency. If they are too small, the liquid permeability during filtration of the crystals may be lowered.

Further, the LW ratio (length to width ratio) and average LW ratio in the particle size distribution ($D_{10}$, $D_{50}$, $D_{90}$) of the 3-hydroxypropionic acid salt crystal are 0.50 or more and 3.00 or less, 0.70 or more, 2.80 or less, and 1.00 or more and 2.50 or less. If the LW ratio of the 3-hydroxypropionic acid salt crystals is too large, problems of fluidity and clogging may occur during transfer of the crystals, and if the LW ratio is too small, the liquid permeability during filtration of the crystals may be lowered.

The 3-hydroxypropionic acid salt crystal can measure the moisture content contained in the crystals by the Karl Fischer method, and the moisture content contained in the 3-hydroxypropionic acid salt crystal may be 200 ppm or more and 5000 ppm or less, 250 ppm or more and 4800 ppm or less, 300 ppm or more and 4600 ppm or less, or 350 ppm or more and 4400 ppm or less.

At this time, the moisture contained in the 3-hydroxypropionic acid salt crystal means the attached moisture contained between the crystals, rather than the crystal moisture (for example, $Ca(3HP)_2 \cdot 2H_2O$). Further, if the moisture content contained in the 3-hydroxypropionic acid salt crystal is too high, it may be recovered in the form of a slurry rather than a crystalline solid, or impurities may be contained in the water, which may cause a problem of a decrease in purity improvement.

In the recovery process according to one embodiment of the invention, as 3-hydroxypropionic acid is prepared through a process such as fermenting a bacterium strain having 3-hydroxypropionic acid-producing ability as described below, the 3-hydroxypropionic acid salt crystal may contain a radioactive carbon isotope ($^{14}C$).

The radioactive carbon isotope ($^{14}C$) contains about 1 atom per $10^{12}$ carbon atoms in earth's atmosphere and has a half-life of about 5700 years, and carbon stocks can become abundant in the upper atmosphere due to nuclear reactions in which cosmic rays and normal nitrogen ($^{14}N$) participate. Meanwhile, in fossil fuels, isotopes have decayed long ago, so that the $^{14}C$ ratio may be substantially zero. When bio-derived raw materials are used as the 3-hydroxypropionic acid raw material, or fossil fuels are used together with it, the content of radioactive carbon isotopes (pMC; percent modern carbon) and the content of bio-carbon contained in 3-hydroxypropionic acid can be measured according to the standard of ASTM D6866-21,

After carbon atoms contained in the compound to be measured is made into graphite form or carbon dioxide gas form, and can be measured, for example, by the mass spectrometer, or can be measured according to liquid scintillation analysis method. At this time, the two radioactive isotopes can be separated using an accelerator for separating $^{14}C$ ions from $^{12}C$ ions together with the mass spectrometer, and the content and the content ratio can be measured by a mass spectrometer.

The 3-hydroxypropionic acid salt crystal has a radiocarbon isotope content of 20 pMC (percent modern carbon) or more, 50 pMC or more, 90 pMC or more, 100 pMC or more, and a biocarbon content of 20 wt.% or more, 50 wt.% or more, 80 wt.%, 90 wt.%, or 95 wt.%, as measured according to the standard of ASTM D6866-21.

The radiocarbon isotope ratio (pMC) means the ratio of the radiocarbon isotope ($^{14}C$) contained in the 3-hydroxypropionic acid crystals to the radiocarbon isotope ($^{14}C$) of the current standard reference material, and it can be greater than 100% because the nuclear test program of the 1950s is still in effect and not extinguished.

Further, the content of biocarbon means the content of biocarbon relative to the total carbon content contained in the 3-hydroxypropionic acid salt crystal. As this value is larger, it may correspond to an environmentally friendly compound.

Meanwhile, if the radiocarbon isotope content (pMC) and biocarbon content of the 3-hydroxypropionic acid salt crystal are too low, the environmental friendliness is reduced, which may not be seen as a bio-derived material.

The crystalline state of the 3-hydroxypropionic acid salt crystal can be confirmed through peaks in an X-ray diffraction (XRD) graph.

For example, the 3-hydroxypropionic acid salt crystal may exhibit peaks between crystal lattices in the $2\theta$ value range of 8 to 22° during X-ray diffraction (XRD) analysis.

For example, when the concentrate contains magnesium hydroxide ($Mg(OH)_2$), and the formed 3-hydroxypropionic acid salt crystal is $Mg(3HP)_2$, peaks between the crystal lattices due to the bond between 3-hydroxypropionic acid and magnesium may appear in the $2\theta$ value range of 8 to 15° during X-ray diffraction (XRD) analysis for $Mg(3HP)_2$. Such peaks show different results from the X-ray diffraction (XRD) analysis results for magnesium hydroxide ($Mg(OH)_2$) or magnesium sulfate ($Mg(SO_4)$). As a result of the X-ray diffraction (XRD) analysis, it can be confirmed that when a specific peak appears in the $2\theta$ value range of 8 to 15°, $Mg(3HP)_2$ crystal is formed.

Specifically, during X-ray diffraction (XRD) analysis for $Mg(3HP)_2$, 3 or more, 4 or more, or 5 or more peaks may appear in the $2\theta$ value range of 8 to 15°, and for example, peaks may appear in the $2\theta$ value range of 8.2 to 9.3°, 9.5 to 11.0°, 11.2 to 12.7°, 12.9 to 13.3°, and 13.5 to 14.8°, respectively.

Further, when calcium hydroxide ($Ca(OH)_2$) is contained in the concentrate and the 3-hydroxypropionic acid salt crystal formed therefrom are $Ca(3HP)_2$, peaks between the crystal lattices due to the bond between 3-hydroxypropionic acid and calcium may appear in the $2\theta$ value range of 10 to 22° during X-ray diffraction (XRD) analysis for $Ca(3HP)_2$. Such peaks show different results from the X-ray diffraction (XRD) analysis results for calcium hydroxide ($Ca(OH)_2$) or calcium sulfate ($Ca(SO_4)$). As a result of the X-ray diffraction (XRD) analysis, it can be confirmed that when a specific peak appears in the $2\theta$ value range of 10 to 22°, $Ca(3HP)_2$ crystal is formed.

Specifically, during X-ray diffraction (XRD) analysis for $Ca(3HP)_2$, 3 or more, 5 or more, 7 or more or 9 or more peaks may appear in the $2\theta$ value range of 10 to 22°, and for example, peaks may appear in the $2\theta$ value range of 10.0 to 11.0°, 11.1 to 11.6°, 11.6 to 12.5°, 12.7 to 13.6°, 13.8 to 16.0°, 17.0 to 18.0°, 19.0 to 19.8°, 20.2 to 21.2°, or 21.5 to 22.0°, respectively.

Meanwhile, the incident angle (8) means the point at which a first differential value (slope of the tangent line, dy/dx) is 0 on a graph where the horizontal axis (x-axis) in the x-y plane is a value of two times the incident angle ($2\theta$) of the

incident X-ray, and the vertical axis (y-axis) in the x-y plane is a diffraction intensity, wherein as the value (2θ) of two times the angle of incidence of the incident X-ray, which is the horizontal axis (x-axis), increases in the positive direction, the first differential value of two times (2θ) the incident angle of X-rays, which is the horizontal axis (x-axis), with respect to the diffraction intensity, which is the vertical axis (y-axis), changes from a positive value to a negative value.

Further, the 3-hydroxypropionic acid salt crystal may have a distance (d value) between atoms in the crystals derived from X-ray diffraction (XRD) analysis of 1.00 Å or more and 15.00 Å or less, 1.50 Å or more and 13.00 Å or less, 2.00 Å or more and 11.00 Å or less, 2.50 Å or more and 10.00 Å or less.

For example, when the 3-hydroxypropionic acid salt crystal is $Mg(3HP)_2$, a distance (d value) between atoms in the crystals of the peak appearing in the 2θ value range of 8 to 15° may be 1.00 Å or more and 15.00 Å or less, 2.00 Å or more and 13.00 Å or less, 4.00 Å or more and 11.00 Å or less, 5.50 Å or more and 10.00 Å or less.

Further, when the 3-hydroxypropionic acid salt crystal is $Ca(3HP)_2$, the distance (d value) between atoms in the crystals of the peak appearing in the 2θ value range of 10 to 22° may be 1.00 Å or more and 15.00 Å or less, 2.00 Å or more and 13.00 Å or less, 3.00 Å or more and 10.00 Å or less, 3.40 Å or more and 9.00 Å or less, or 4.00 Å or more and 8.50 Å or less.

Further, the 3-hydroxypropionic acid salt crystal may have a glass transition temperature of -55°C or more and -30°C or less, a melting point of 30°C or more and 170°C or less, and a crystallization temperature of 25°C or more and 170°C or less.

The glass transition temperature, melting point, and crystallization temperature may be measured by a differential scanning calorimetry (DSC) for the 3-hydroxypropionic acid salt crystal, wherein the heating rate during measurement may be 1 to 20°C/min. Further, the 3-hydroxypropionic acid salt crystal ate may have a glass transition temperature of -55°C or more and -30°C or less, -50°C or more and -35°C or less, or -45°C or more and -40 °C or less. Further, the melting point of the 3-hydroxypropionic acid salt crystal may be 30°C or more and 170°C or less, 31°C or more and 160°C or less, 32°C or more and 150°C or less. Further, the crystallization temperature of the 3-hydroxypropionic acid salt crystal may be 25°C or more and 170 °C or less, 27°C or more and 160°C or less, or 30°C or more and 150°C or less. In addition, the crystallization stability section of the 3-hydroxypropionic acid salt crystal may be -40°C to 150°C.

The purity of 3-hydroxypropionic acid salt contained in the 3-hydroxypropionic acid salt crystal may be calculated as a percentage (%) of the mass of the compound having the structural formula 1 and/or structural formula 2 relative to the mass of the total crystal recovered. For example, the purity of 3-hydroxypropionic acid salt contained in the 3-hydroxypropionic acid salt crystal may be 70% or more, 80% or more, 90% or more, 70 to 99.9%, 80 to 99.9%, 90 to 99.9%, 70 to 99%, 80 to 99%, or 90 to 99%, but is not limited thereto.

The recovery process according to one embodiment of the invention may comprise fermenting a bacterium strain having 3-hydroxypropionic acid-producing ability to produce a 3-hydroxypropionic acid fermentation liquor, and concentrating the fermentation liquid to form a concentrate containing 300 g/L of more of 3-hydroxypropionic acid, prior to the step of forming the 3-hydroxypropionic acid salt crystal.

The bacterium strain having 3-hydroxypropionic acid-producing ability may include genes encoding at least one or two proteins selected from the group consisting of glycerol dehydratase and aldehyde dehydrogenase.

In one example, the 3-hydroxypropionic acid-producing strain may further include a gene (gdrAB) encoding glycerol dehydratase reactivator (GdrAB). In one example, the 3-hydroxypropionic acid-producing strain may be a bacterium strain that is further capable of biosynthesizing vitamin B12.

The glycerol dehydratase may be encoded by dhaB (GenBank accession no. U30903.1) gene, but is not limited thereto. The dhaB gene may be an enzyme derived from *Klebsiella pneumonia,* but is not limited thereto. The gene encoding the glycerol dehydratase may include a gene encoding dhaB1, dhaB2 and/or dhaB3. The glycerol dehydratase protein and the gene encoding it may include mutations in the gene and/or amino acid sequence within the range of maintaining an enzyme activity that decomposes glycerol to 3-hydroxypropanal (3-HPA) and water ($H_2O$).

The gene (aldH) encoding the aldehyde dehydrogenase (ALDH) may be, for example, aldH (GenBank Accession no. U00096.3; EaldH) gene derived from *Escherichia coli* or E. *coli* K12 MG1655 cell line, puuC gene derived from *Klebsiella pneumoniae,* and/or KGSADH gene from *Azospirillum brasilense,* but is not limited thereto. The aldehyde dehydrogenase protein and the gene encoding it may include mutations in the gene and/or amino acid sequence within the range of maintaining the activity for producing 3-hydroxypropionic acid from 3-hydroxypropanal.

The medium for producing the fermentation liquid can be selected without limitation within the range for the purposes for producing 3-hydroxypropionic acid. In one example, the medium may contain glycerol as a carbon source. In another example, the medium may be crude glycerol and/or pretreated crude glycerol, but is not limited thereto. In one example, the production medium may further include vitamin B12.

In the step of fermenting a bacterium strain having 3-hydroxypropionic acid-producing ability to produce a 3-hydroxypropionic acid fermentation liquor, the concentration of 3-hydroxypropionic acid contained in the 3-hydroxypropionic acid fermentation liquor may be 1 to 200 g/L, 10 to 150 g/L, 30 to 130 g/L, or 40 to 100 g/L.

Further, the fermentation may be a neutral fermentation, for example, it may be maintained in the pH range of 6 to 8, 6.5 to 8, 6 to 7.5, or 6.5 to 7.5 during fermentation, but is not limited thereto. The pH range can be appropriately adjusted as needed. The alkali metal salt or alkali earth metal salt may be added for the neutral fermentation. The alkali metal salt or alkali earth metal salt may include $Mg^{2+}$, $Ca^{2+}$ or a mixture thereof. Further, the alkali metal salt or alkali earth metal salt may be $Ca(OH)_2$ or $Mg(OH)_2$ , but is not limited thereto.

The recovery process according to one embodiment of the invention may further include removing (separating) cells from the fermentation liquid; purifying and/or decolorizing the fermentation liquid and/or the fermentation liquid from which cells has been removed; and/or filtering the fermentation liquid and/or the fermentation liquid from which cells have been removed, after the step of producing the 3-hydroxypropionic acid fermentation liquid.

Removal (separation) of the cells may be carried out by selecting methods known in the art without limitation within the range of cell (strain) removal purposes. In one example, the separation of the cells may be carried out by centrifugation.

The step of purifying and/or decolorizing the fermentation liquid and/or the fermentation liquid from which cells have been removed can be carried out by selecting methods known in the art without limitation within the range of the purpose of purification of the fermentation liquid. For example, the step can be carried out by mixing activated carbon with the fermentation liquid and then removing the activated carbon, but is not limited thereto.

The step of filtering the fermentation liquid and/or the fermentation liquid from which cells have been removed can be carried out by selecting methods known in the art without limitation within the range of the purposes of removal of solid impurities, removal of proteins and/or materials with hydrophobic functional groups, and/or decoloration. For example, the step can be carried out by filter filtration and/or activated carbon filtration methods, but is not limited thereto.

The recovery process according to one embodiment of the invention may comprise concentrating the fermentation liquid to form the concentrate containing 3-hydroxypropionic acid, after the step of fermenting the bacterium strain having 3-hydroxypropionic acid-producing ability to produce a 3-hydroxypropionic acid fermentation liquid.

Concentration of the fermentation liquid may be carried out by evaporating the fermentation liquid (e.g., a liquid component of the fermentation liquid).

The concentration may be carried out by any means commonly available for evaporating the liquid component of the fermentation liquid. For example, the concentration may be carried out by rotary evaporation, evaporation concentration, vacuum concentration, and reduced pressure concentration, but is not limited thereto.

In one example, the concentration of 3-hydroxypropionic acid in the fermentation liquid after concentration may be increased by 2 to 50 times, 2 to 40 times, 2 to 30 times, 2 to 20 times, 2 to 10 times, 5 to 50 times, 5 to 40 times, 5 to 30 times, 5 to 20 times, or 5 to 10 times compared to before concentration.

In addition, the recovery process according to one embodiment of the invention may further comprise reacting the alkali metal salt hydrate or alkali earth metal salt hydrate with ammonia water to form an alkali metal hydroxide or alkali earth metal hydroxide.

The present inventors have found through experiments that by reacting the alkali metal salt hydrate or alkali earth metal salt hydrate with ammonia water, the alkali metal hydroxide or alkali earth metal hydroxide that can be recycled for various purposes can be recovered at a high recovery rate, and completed the invention.

Anhydride formed by drying an alkali metal salt hydrate or alkali earth metal salt hydrate such as $CaSO_4 \cdot 2H_2O$ can be reacted with ammonia water to form an alkali metal hydroxide or alkali earth metal hydroxide. For example, the anhydride may be calcium sulfate ($CaSO_4$), and calcium sulfate and ammonia water react as shown in the following Reaction Formula 2 to form an alkali metal hydroxide or alkali earth metal hydroxide such as $Ca(OH)_2$ and ammonium sulfate.

$$[\text{Reaction Scheme 2}] \qquad CaSO_4 + 2NH_3 \cdot H_2O \rightarrow Ca(OH)_2 + (NH_4)_2SO_4$$

An alkali metal hydroxide or alkali earth metal hydroxide such as $Ca(OH)_2$ is reused as an alkali metal salt or alkali earth metal salt in the step of forming 3-hydroxypropionic acid salt crystal, or can be reused as a neutralizing agent in the step of fermenting a bacterium strain having 3-hydroxypropionic acid-producing ability, thereby carrying out neutral fermentation.

Further, the ammonium sulfate can be recycled as ammonium sulfite fertilizer through processes such as concentration and drying. Therefore, the recovery process according to one embodiment of the invention reduces the generation of waste and thus is environmentally friendly, and can be economically constructed by reusing the alkali metal hydroxide or alkali earth metal hydroxide.

In addition, the recovery process may further comprise separating the alkali metal salt hydrate or alkali earth metal salt hydrate formed in the step of adding the acid and then drying the separated alkali metal salt hydrate or alkali earth metal salt hydrate. By drying the alkali metal salt hydrate or alkali earth metal salt hydrate , the alkali metal salt anhydride or alkali earth metal salt anhydride such as gypsum anhydrite can be recovered.

Therefore, the ammonia water may be reacted with the alkali metal salt hydrate or alkali earth metal salt hydrate, or reacted with the dried alkali metal salt anhydride or alkali earth metal salt anhydride.

The reaction between the hydrate or anhydride of the alkali metal salt or alkali earth metal salt and ammonia water is not limited thereto, but can be carried out by adding ammonia water to a solution containing the hydrate or anhydride of the alkali metal salt , or alkali earth metal salt or can be carried out by adding the hydrate or anhydride of the alkali metal salt or alkali earth metal salt to the ammonia water.

By reacting the alkali metal or alkali earth metal salt hydrate or anhydride with the ammonia, an ammonium salt can be further formed in addition to the alkali metal hydroxide or alkali earth metal hydroxide. For example, the ammonium salt may be ammonium sulfate $((NH_4)_2SO_4)$.

Further, the ammonium sulfate can be recycled as a fertilizer. Specifically, after the step of concentrating and/or drying the formed ammonium sulfate, it can be used as an ammonium sulfite fertilizer.

In the step of forming the alkali metal hydroxide or alkali earth metal hydroxide, the equivalent of ammonia used relative to the total equivalent of the hydrate or anhydride of the alkali metal or alkali earth metal can be 1 time or more and 50 times or less, 5 times or more and 40 times or less, 10 times or more and 30 times or less. When the amount of ammonia used is too small, the conversion rate from the hydrate or anhydride of the alkali metal salt or alkali earth metal salt to the alkali metal hydroxide or alkali earth metal hydroxide is low, and the content of the alkali metal hydroxide or alkali earth metal hydroxide that can be recovered and reused may be small.

In addition, the recovery process according to one embodiment of the invention may further comprise adding the alkali metal salt hydrate or alkali earth metal salt hydrate to a hydroxide ion ($OH^-$)-containing solution to form an alkali metal hydroxide or alkali earth metal hydroxide.

The present inventors have found through experiments that by adding the alkali metal salt hydrate or alkali earth metal salt hydrate to a solution containing hydroxide ion ($OH^-$), the alkali metal hydroxide or alkali earth metal hydroxide that can be recycled for various purposes can be recovered at a high recovery rate, and completed the invention.

An anhydride formed by drying an alkali metal salt hydrate or alkali earth metal salt hydrate such as $CaSO_4 \cdot 2H_2O$ can be added to a hydroxide ion ($OH^-$)-containing solution to form an alkali metal hydroxide or alkali earth metal hydroxide. For example, the anhydride may be calcium sulfate ($CaSO_4$), and the hydroxide ion ($OH^-$)-containing solution may be a solution containing sodium hydroxide, and such calcium sulfate and sodium hydroxide may form an alkali metal hydroxide or alkali earth metal hydroxide such as $Ca(OH)_2$ through a reaction shown in the following Reaction Formula 3.

$$[\text{Reaction Scheme 3}] \qquad CaSO_4 + NaOH \rightarrow Ca(OH)_2 + Na_2SO_4$$

An alkali metal hydroxide or alkali earth metal hydroxide such as $Ca(OH)_2$ is reused as an alkali metal salt or alkali earth metal salt in the step of forming 3-hydroxypropionic acid salt crystal, or can be reused as a neutralizing agent in the step of fermenting a bacterium strain having 3-hydroxypropionic acid-producing ability, thereby carrying out neutral fermentation.

In addition, the recovery process may further comprise separating the alkali metal salt hydrate or alkali earth metal salt hydrate formed in the step of adding the acid, and then drying the separated alkali metal salt hydrate or alkali earth metal salt hydrate . By drying the alkali metal salt hydrate or alkali earth metal salt hydrate, the alkali metal salt anhydride or alkali earth metal salt anhydride such as gypsum anhydrite can be recovered.

Therefore, the hydroxide ion ($OH^-$)-containing solution can be charged with the alkali metal salt hydrate or alkali earth metal salt hydrate , or charged together with the dried alkali metal salt anhydride or alkali earth metal salt anhydride.

Further, the hydroxide ion ($OH^-$)-containing solution is not particularly limited as long as it is a solution containing hydroxide ion ($OH^-$), but for example, the hydroxide ion ($OH^-$)-containing solution may include one or more selected from the group consisting of sodium hydroxide (NaOH), potassium hydroxide (KOH), barium hydroxide ($Ba(OH)_2$), lithium hydroxide (LiOH), rubidium hydroxide (RbOH), cesium hydroxide (CsOH), calcium hydroxide ($Ca(OH)_2$) and strontium hydroxide ($Sr(OH)_2$).

In the step of forming the alkali metal hydroxide or alkali earth metal hydroxide, the equivalent of hydroxide ion used relative to the total equivalent of the hydrate or anhydride of the alkali metal salt or alkali earth metal salt may be 1 time or more and 20 times or less, 2 times or more and 15 times or less, 3 times or more and 10 times or less. When the equivalent of the hydroxide ion used is too small, the conversion rate from the hydrate or anhydride of the alkali metal salt or alkali earth metal salt to the alkali metal hydroxide or alkali earth metal hydroxide is low, and the content of the hydroxide of the alkali metal or alkali earth metal that can be recovered and reused may be small.

The recovery rate of the alkali metal hydroxide or alkali earth metal hydroxide may be 40% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, but is not limited thereto. The recovery rate may be calculated based on the weight.

The alkali metal hydroxide or alkali earth metal hydroxide can be reused as the alkali metal salt or alkali earth metal salt in the step of forming and separating 3-hydroxypropionic acid salt crystal from the concentrate containing 3-hydroxypropionic acid.

Further, the alkali metal hydroxide or alkali earth metal hydroxide may be reused during the fermentation, thereby carrying out neutral fermentation.

Further, the recovery process according to one embodiment of the invention may further comprise reacting a hydrate of the alkali metal salt or alkali earth metal salt with a carbonate to form an alkali metal carbonate or alkali earth metal carbonate.

The present inventors have found through experiments that by reacting the hydrate of the alkali metal salt or alkali earth metal salt with a carbonate, the alkali metal carbonate or alkali earth metal carbonate that can be recycled for various purposes can be recovered at a high recovery rate, and completed the invention.

The anhydride formed by drying the hydrate of an alkali metal salt or alkali earth metal salt such as $CaSO_4 \cdot 2H_2O$ can be reacted with a carbonate to form an alkali metal carbonate or alkali earth metal carbonate. For example, the anhydride may be calcium sulfate ($CaSO_4$), and the carbonate may be ammonium carbonate ($(NH_4)_2CO_3$). These can form an alkali metal carbonate or alkali earth metal carbonate such as $CaCO_3$ and ammonium sulfate by the reaction shown in the following Reaction Formula 4.

$$[\text{Reaction Scheme 4}] \qquad CaSO_4 + (NH_4)_2CO_3 \rightarrow CaCO_3 + (NH_4)_2SO_4$$

The alkali metal carbonate or alkali earth metal carbonate such as $CaCO_3$ may be reused as an alkali metal salt or alkali earth metal salt in the step of forming 3-hydroxypropionic acid salt crystal, or may be reused as a neutralizing agent in the step of fermenting a bacterium strain having 3-hydroxypropionic acid-producing ability, thereby carrying out neutral fermentation.

Further, the ammonium sulfate can be recycled as ammonium sulfite fertilizer through processes such as concentration and drying. Therefore, the recovery process according to one embodiment of the invention reduces the generation of waste and thus is environmentally friendly, and can be economically constructed by reusing the alkali metal carbonate or alkali earth metal carbonate.

In addition, the recovery process may further comprise separating the alkali metal salt hydrate or alkali earth metal salt hydrate formed in the step of adding the acid, and then drying the separated alkali metal salt hydrate or alkali earth metal salt hydrate. By drying the alkali metal salt hydrate or alkali earth metal salt hydrate, the alkali metal salt anhydride or alkali earth metal salt anhydride such as gypsum anhydrite can be recovered.

Therefore, the carbonate may be reacted with the alkali metal salt hydrate or alkali earth metal salt hydrate, or reacted with the dried alkali metal salt anhydride or alkali earth metal salt anhydride.

The reaction between the hydrate or anhydride of the alkali metal salt or alkali earth metal salt and the carbonate is not limited thereto, but can be carried out by adding a carbonate to a solution containing the hydrate or anhydride of the alkali metal salt or alkali earth metal salt, or can be carried out by adding the hydrate or anhydride of the alkali metal salt or alkali earth metal salt to the solution containing carbonate.

Further, the carbonate is not particularly limited as long as it is a compound containing carbonate ion ($CO_3^{2-}$), but for example, it may be ammonium carbonate ($(NH_4)_2CO_3$).

By reacting the alkali metal or alkali earth metal salt hydrate or anhydride with the ammonium carbonate, an ammonium salt can be further formed in addition to the alkali metal carbonate or alkali earth metal carbonate. For example, the ammonium salt may be ammonium sulfate ($(NH_4)_2SO_4$).

Further, the ammonium sulfate can be recycled as fertilizer. Specifically, after the step of concentrating and/or drying the formed ammonium sulfate, it can be used as an ammonium sulfite fertilizer.

In the step of forming the alkali metal carbonate or alkali earth metal carbonate, the equivalent of carbonate used relative to the total equivalent of the hydrate or anhydride of the alkali metal salt or alkali earth metal salt can be 1 time or more and 20 times or less, 2 times or more and 15 times or less, 5 times or more and 10 times or less. When the amount of carbonate used is too small, the conversion rate from the hydrate or anhydride of the alkali metal salt or alkali earth metal salt to the alkali metal hydroxide or alkali earth metal hydroxide is low, and the content of the alkali metal hydroxide or alkali earth metal hydroxide that can be recovered and reused may be small.

Further, the recovery rate of the alkali metal carbonate or alkali earth metal carbonate may be 40% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, but is not limited thereto. The recovery rate may be calculated based on the weight.

The alkali metal carbonate or alkali earth metal carbonate can be reused as the alkali metal salt or alkali earth metal salt in the step of forming and separating 3-hydroxypropionic acid salt crystal from the concentrate containing 3-hydroxypropionic acid.

Further, the alkali metal carbonate or alkali earth metal carbonate may be reused during the fermentation, thereby carrying out neutral fermentation.

According to another embodiment of the present invention, a 3-hydroxypropionic acid-containing slurry composition comprising a precipitate represented by the following structural formula 4 and 3-hydroxypropionic acid can be provided.

[Structural Formula 4]     Cation(Anion)·pH$_2$O

in the structural formula 4, the Cation is the cation of the alkali metal salt or alkali earth metal salt, and for example, it may be Na$^+$, Mg$^{2+}$ or Ca$^{2+}$, but when it is Mg$^{2+}$ or Ca$^{2+}$, 3-hydroxypropionic acid salt crystal can be formed more effectively.

Further, the Anion is an anion of the acid, and may be, for example, SO$_4$$^{2-}$, PO$_4$$^{3-}$or CO$_3$$^{2-}$, but is not limited thereto. The p is the number of water molecules in a hydrate, and is an integer of 1 or more.

The slurry composition may be formed in the recovery process according to one embodiment of the invention, and for example, it may be formed in the step of adding an acid to 3-hydroxypropionic acid salt crystal separated from the concentrate at a temperature of, for example, 0°C or more and 90°C or less, 15°C to 90°C, or 30°C or more and 90°C or less.

The particle size of the precipitate may be 0.5 $\mu$m or more, 1.0 $\mu$m or more, 1.5 $\mu$m or more, and may be 500.0 $\mu$m or less, 400.0 $\mu$m or less, 300.0 $\mu$m or less, 200.0 $\mu$m or less, 100 $\mu$m or less, 80 $\mu$m or less, 50 $\mu$m or less, 30 $\mu$m or less, 20 $\mu$m or less, 8.0 $\mu$m or less, 7.0 $\mu$m or less, 6.0 $\mu$m or less, 5.0 $\mu$m or less. The precipitate satisfies the above-mentioned particle size, and thus, the moisture content of the precipitate can be lowered, which can increase the fluidity of a slurry containing the precipitate and facilitate filtration of the precipitates from the slurry. The particle size of the precipitate can be measured by a scanning electron microscopy (SEM).

The precipitate may exhibit various particle shapes such as angular, spherical, plate-shape, and needle-shape. At this time, the particle size of the precipitate is measured based on the straight-line distance between crystal planes with the longest distance among the straight-line distances between crystal planes contained in the precipitate.

In addition, the moisture content of the precipitate may be 150% or less, 130% or less, 110% or less, 100% or less, 80% or less, 70% or less, and 1% or more, 10% or more, 30% or more. If the moisture content of the precipitate is too high, the fluidity of the slurry containing the precipitate is lowered, which makes it difficult to proceed with the process, and makes it difficult to separate the precipitate from the slurry, thus making it difficult to recover 3-hydroxypropionic acid in high yield.

## ADVANTAGEOUS EFFECTS

According to the present invention, there can be provided a method of efficiently separating and recovering 3-hydroxypropionic acid derived from an environmentally friendly bioprocess in a high purity state, and at the same time, efficiently recovering alkali metal salt hydrate or alkali earth metal salt hydrate that can be recycled for various purposes.

## BRIEF DESCRIPTION OF THE DRAWINGS

FIG. 1 shows the results of X-ray diffraction analysis of the precipitate formed in Example 1.

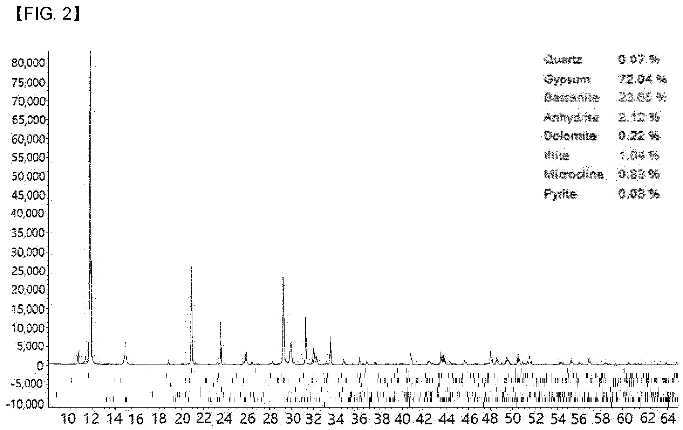

FIG. 2 shows the results of X-ray diffraction analysis of the precipitate formed in Example 2.

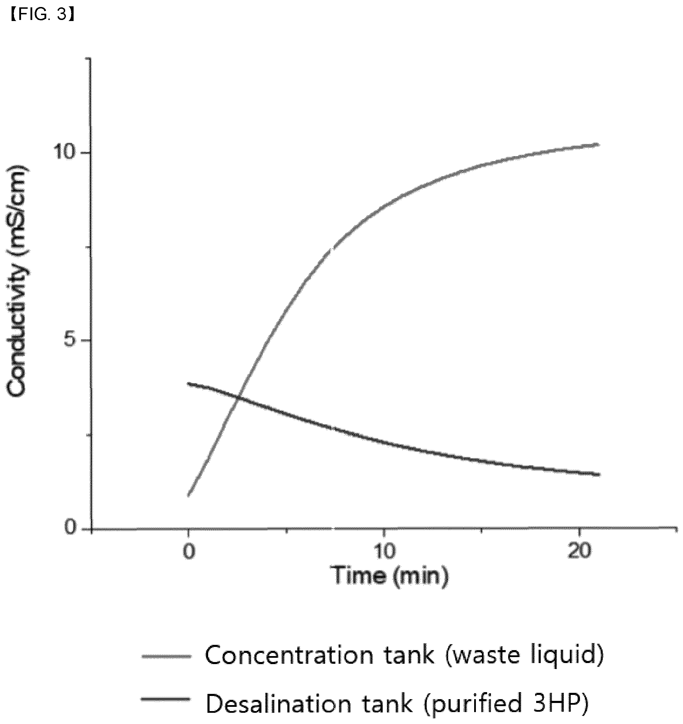

FIG. 3 is a graph showing the amount of change in electrical conductivity over time in the electrodialysis step of Example 4.
FIG. 4 shows the results of X-ray diffraction analysis of solid A of Example 6.
FIG. 5 shows the results of standard X-ray diffraction analysis of calcium hydroxide.
FIG. 6 shows the results of X-ray diffraction analysis of solid A of Example 7.
FIG. 7 shows the results of X-ray diffraction analysis of solid B of Example 7.
FIG. 8 shows the results of X-ray diffraction analysis of solid A of Example 8.
FIG. 9 shows the results of standard X-ray diffraction analysis of calcium carbonate.
FIG. 10 shows the results of X-ray diffraction analysis of solid B of Example 8.
FIG. 11 is a graph overlapping X-ray diffraction analysis results of solid A, calcium carbonate and solid B of Example 8.

## DETAILED DESCRIPTION OF THE EMBODIMENTS

Hereinafter, the present invention will be described in more detail with reference to examples. However, the following examples are for illustrative purposes only, and are not intended to limit the scope of the present invention.

**Preparation Example 1: Preparation of bacterium strain for producing 3-hydroxypropionic acid**

Recombinant vectors into which genes encoding glycerol dehydratase and aldehyde dehydrogenase, which are known to produce 3-hydroxypropionic acid (3HP) using glycerol as a substrate, were introduced were manufactured. The prepared recombinant vector was introduced into *E.coli* W3110 strain to prepare a 3-hydroxypropionic acid-producing strain.

More specifically, a BtuR gene encoding adenosyltransferase was cloned into plasmid pCDF containing a gene (dhaB) encoding glycerol dehydratase, a gene (aldH) encoding aldehyde dehydrogenase and a gene (gdrAB) encoding glycerol dehydratase reactivase. The resulting pCDF_J23101_dhaB_gdrAB_J23100_aldH_btuR vector was introduced into strain W3110 (KCCM 40219) by an electroporation method using an electroporation device (Bio-Rad, Gene Pulser Xcell) to prepare 3-hydroxypropionic acid-producing strain. The process of preparing the 3-hydroxypropionic acid-producing strain of Preparation Example 1 and the vectors, primers, and enzymes used were carried out with reference to Example 1 of Korean Unexamined Patent Publication No. 10-2020-0051375,

**Preparation Example 2: Preparation of Ca(3HP)$_2$ Crystal**

The 3-hydroxypropionic acid-producing strain prepared in Preparation Example 1 was fermented and cultured at 35°C in a 5L fermenter using unpurified glycerol as a carbon source to produce 3-hydroxypropionic acid. In order to prevent the lowering of pH due to the production of 3-hydroxypropionic acid, calcium hydroxide (Ca(OH)$_2$), which is an alkali metal salt or alkali earth metal salt, was added to keep the neutral pH during the fermentation.

After fermented culture, cells were removed by centrifugation (4000 rpm, 10 minutes, 4 °C), and primary fermentation liquid purification (primary purification) was performed using activated carbon. Specifically, activated carbon was added to the fermentation liquid from which cells were removed by centrifugation, the mixture was well mixed, and then centrifuged again to separate the activated carbon. Then, the fermentation liquid from which the activated carbon was separated was filtered with a vacuum pump through a 0.7 um filter paper to purify the 3-hydroxypropionic acid fermented liquid.

The concentration of 3-hydroxypropionic acid in the fermentation liquid after the primary purification was at a level of 50 to 100 g/L, and the fermentation liquid was concentrated to a concentration of 600 g/L using a rotary evaporator (50°C, 50 mbar) to prepare a concentrate, and ethanol was added in an amount of two times the volume of the concentrate, and stirred (300 rpm) at room temperature to produce Ca(3HP)$_2$ crystals. At this time, the concentration of the alkali metal salt or alkali earth metal salt in the concentrate was 493.3 g/L (based on Ca(OH)$_2$). The resulting crystals were washed three times with ethanol (EtOH) and dried in an oven at 50°C to finally recover the crystals.

**Example 1**

An aqueous solution containing 600 g/L of Ca(3HP)$_2$ crystal recovered in Preparation Example 2 was prepared, and stirred at a temperature of 25°C and 350 rpm for 10 minutes.

37 ml of 5M sulfuric acid solution was added to 77 ml of the aqueous solution at a uniform rate for 5 minutes, and further stirred for 30 minutes to form a slurry containing CaSO$_4$ precipitate and 3-hydroxypropionic acid.

Filtration was performed using a filtration flask and a vacuum pump in order to separate the CaSO$_4$ precipitate. Then, a filtrate (A) before washing was obtained in a filtering flask, and the filtered CaSO$_4$ precipitate was washed with 30 ml of distilled water, and then filtered to obtain a filtrate (B) after washing. Then, the CaSO$_4$ precipitate was dried in an oven at a temperature of 60°C for 20 hours to finally obtain the dried CaSO$_4$ precipitate. In addition, filtrates (A) and (B) containing 3-hydroxypropionic acid were obtained.

**Example 2**

An aqueous solution containing 150 g/L of Ca(3HP)$_2$ crystal recovered in Preparation Example 2 was prepared, and heated to a temperature of 60°C while stirring at 350 rpm.

250 ml of 2.72M sulfuric acid solution was added to 1 L of the aqueous solution at a uniform rate for 60 minutes, and further stirred for 30 minutes to form a slurry containing CaSO$_4$ precipitate and 3-hydroxypropionic acid, which was further stirred until the temperature dropped to room temperature.

Filtration was performed using a filtration flask and a vacuum pump in order to separate the CaSO$_4$ precipitate. Then, a filtrate (A) before washing was obtained in a filtering flask, and the filtered CaSO$_4$ precipitate was washed with 100 ml of distilled water, and then filtered to obtain a filtrate (B) after washing. Then, the CaSO$_4$ precipitate was dried in an oven at a temperature of 60°C for 20 hours to finally obtain the dried CaSO$_4$ precipitate. In addition, filtrates (A) and (B) containing 3-hydroxypropionic acid were obtained.

**Example 3**

A cation exchange resin column was filled with 12 BV of cation exchange resin, and then washed with water to prepare a cation exchange resin column. At this time, TRILITE®CMP28LH (Samyang Corporation) was used as a cation exchange resin.

The entire filtrate containing 3-hydroxypropionic acid obtained in Example 2 was charged into a cation exchange resin column at a space velocity (SV) of 2.4, and then distilled water was charged into to the cation exchange resin column at a space velocity (SV) of 7.2 or less to obtain a 3-hydroxypropionic acid aqueous solution with a pH of 2.5 or less.

### Example 4

An aqueous solution containing 300 g/L of $Ca(3HP)_2$ crystal recovered in Preparation Example 2 was prepared, and stirred at a temperature of 60°C and 350 rpm for 10 minutes.

5M sulfuric acid solution was added to the aqueous solution at a uniform rate for 5 minutes so that the equivalents of 3-hydroxypropionic acid and sulfuric acid were 1:1, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. Then, after obtaining the filtrate (A) before washing in the filtration flask, the filtered $CaSO_4$ precipitate was washed with 30 ml of distilled water and then filtered to obtain a filtrate (B) after washing. Then, the $CaSO_4$ precipitate was dried in an oven at 40°C for 20 hours to finally obtain the dried $CaSO_4$ precipitate.

The obtained filtrate (B) was electrodialyzed using an electrodialysis device (Innomeditech Inc.) Specifically, an electrodialysis tank of the electrodialysis device used L3 membrane (Innomeditech Inc.) as a cation and anion separation membrane, and progressed with a 20-cell constant voltage method (20V, 1V per cell) for 40 minutes. At this time, the desalination tank progressed electrodialysis until the electrical conductivity was lowered to 10% compared to the initial conductivity 100% of the desalination tank, to thereby recover 3-hydroxypropionic acid.

### Example 5

An aqueous solution containing 300 g/L of $Ca(3HP)_2$ crystal recovered in Preparation Example 2 was prepared, and heated to a temperature of 60°C while stirring at 350 rpm.

5M sulfuric acid solution was added to the aqueous solution at a uniform rate for 5 minutes so that the equivalents of 3-hydroxypropionic acid and sulfuric acid were 1:1.2, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. Then, a filtrate (A) before washing was obtained in a filtering flask, and then the filtered $CaSO_4$ precipitate was washed with 30 ml of distilled water, and then filtered to obtain a filtrate (B) after washing. Then, the $CaSO_4$ precipitate was dried in an oven at a temperature of 40°C for 20 hours to finally obtain the dried $CaSO_4$ precipitate.

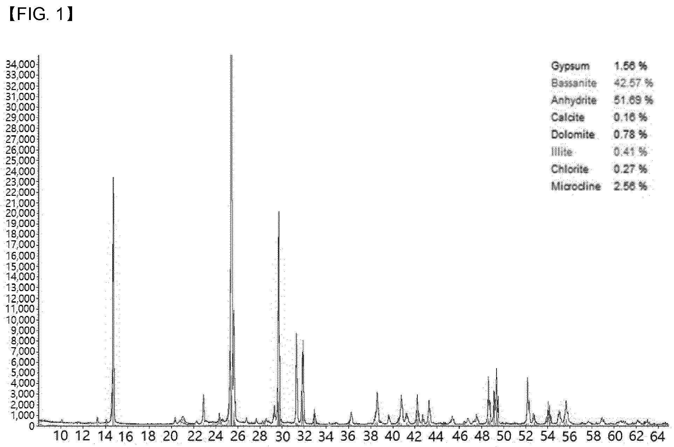

The obtained filtrate (B) was electrodialyzed using an electrodialysis device (Innomeditech Inc.) Specifically, an electrodialysis tank of the electrodialysis device used L3 membrane (Innomeditech Inc.) as a cation and anion separation membrane, and progressed with a 20-cell constant voltage method (20V, 1V per cell) for 40 minutes. At this time, as shown in FIG. 1, the desalination tank progressed electrodialysis until the time point when the electrical conductivity was lowered to 10% compared to the initial conductivity 100% of the desalination tank, to thereby recover 3-hydroxypropionic acid.

### Example 6

An aqueous solution containing 600 g/L of $Ca(3HP)_2$ crystal recovered in Preparation Example 2 was prepared, and stirred at a temperature of 25°C and 350 rpm for 10 minutes.

37 ml of 5M sulfuric acid solution was added to 77 mL of the aqueous solution at a uniform rate for 30 minutes, and further stirred for 30 minutes to form a slurry containing $CaSO_4$ precipitate and 3-hydroxypropionic acid. Filtration was performed using a filtration flask and a vacuum pump in order to separate the $CaSO_4$ precipitate.

The separated $CaSO_4$ precipitate was dried in an oven at a temperature of 80°C for 20 hours to recover gypsum anhydrite($CaSO_4$), which is a solid A. 2.72 g of gypsum anhydrite ($CaSO_4$) was added to 400 mL of 1M ammonia water solution (20 equivalents relative to gypsum anhydrite), and then the mixture was stirred at 200 rpm using a magnetic steel bar while maintaining the temperature at 30°C. After a reaction time of 4 hours has elapsed, the precipitate was filtered using a filtration flask and a vacuum pump to obtain a solid B containing a small amount of moisture. Then, the solid B was dried to recover the dried solid B.

### Example 7

An aqueous solution containing 600 g/L of $Ca(3HP)_2$ crystal recovered in Preparation Example 2 was prepared, and stirred at a temperature of 25°C and 350 rpm for 10 minutes.

37 ml of 5M sulfuric acid solution was added to 77 mL of the aqueous solution at a uniform rate for 5 minutes, and further stirred for 30 minutes to form a slurry containing $CaSO_4$ precipitate and 3-hydroxypropionic acid. Filtration was performed using a filtration flask and a vacuum pump in order to separate the $CaSO_4$ precipitate.

The separated $CaSO_4$ precipitate was dried in an oven at a temperature of 80°C for 20 hours to recover gypsum anhydrite ($CaSO_4$), which is a solid A. 5.44 g of gypsum anhydrite ($CaSO_4$) was added to 200 mL of 1M sodium hydroxide (NaOH) aqueous solution (5 equivalents relative to gypsum anhydrite), and then the mixture was stirred at 200 rpm using a magnetic steel bar while maintaining the temperature at 30°C. After a reaction time of 4 hours has elapsed, the precipitate was filtered using a filtration flask and a vacuum pump to obtain a solid B containing a small amount of moisture. Then, the solid B was dried to recover 2.38 g of the dried solid B.

**Example 8**

An aqueous solution containing 600 g/L of $Ca(3HP)_2$ crystal recovered in Preparation Example 2 was prepared, and stirred at a temperature of 25°C and 350 rpm for 10 minutes.

37 ml of 5M sulfuric acid solution was added to 77 mL of the aqueous solution at a uniform rate for 5 minutes, and further stirred for 30 minutes to form a slurry containing $CaSO_4$ precipitate and 3-hydroxypropionic acid. Filtration was performed using a filtration flask and a vacuum pump in order to separate the $CaSO_4$ precipitate.

The separated $CaSO_4$ precipitate was dried in an oven at a temperature of 80°C for 20 hours to recover gypsum anhydrite ($CaSO_4$), which is a solid A. 4.08 g of gypsum anhydrite ($CaSO_4$) was added to 750 mL of 0.2 M ammonium carbonate (($NH_4)_2CO_3$) aqueous solution (5 equivalents relative to gypsum anhydrite), and then the mixture was stirred at 200 rpm using a magnetic steel bar while maintaining the temperature at 30°C. After a reaction time of 4 hours has elapsed, the precipitate was filtered using a filtration flask and a vacuum pump to obtain a solid B containing a small amount of moisture. Then, the solid B was dried to recover 2.67 g of the dried solid B.

**Example 9**

An aqueous solution containing 250 g/L of $Ca(3HP)_2$ crystal recovered in Preparation Example 2 was prepared, and stirred at a temperature of 60°C and 200 rpm for 10 minutes.

5M phosphoric acid solution was added to the aqueous solution at a uniform rate for 30 minutes so that the equivalents of 3-hydroxypropionic acid and phosphoric acid were 1:1, and further stirred for 30 minutes to form a slurry containing calcium phosphate salt precipitate and 3-hydroxypropionic acid.

Filtration was performed using a filtration flask and a vacuum pump in order to separate the calcium phosphate salt precipitate. Then, a filtrate(A) before washing was obtained in a filtering flask, and the filtered calcium phosphate salt precipitate was washed with 30 ml of distilled water, and then filtered to obtain a filtrate(B) after washing. Then, the calcium phosphate salt precipitate was dried in an oven at a temperature of 80°C for 20 hours to finally obtain the dried calcium phosphate salt precipitate.

A cation exchange resin tower (JCL-GF-F110, JUNG Glass Pyrex) was filled with 12 BV of cation exchange resin, and then washed with water to prepare a cation exchange resin column. At this time, TRILITE®CMP28LH (Samyang Corporation) was used as a cation exchange resin.

The entire filtrate (B) obtained above was charged into a cation exchange resin column at a space velocity (SV) of 2.4 $m^3h^{-1}$, and then distilled water was charged into the cation exchange resin column at a space velocity (SV) of 7.2 $m^3h^{-1}$ or less to obtain a 3-hydroxypropionic acid aqueous solution with a pH of 2.5 or less.

**Example 10**

An aqueous solution containing 250 g/L of $Ca(3HP)_2$ crystal recovered in Preparation Example 2 was prepared, and stirred at a temperature of 25°C and 200 rpm for 10 minutes.

5M phosphoric acid solution was added to the aqueous solution at a uniform rate for 5 minutes so that the equivalents of 3-hydroxypropionic acid and phosphoric acid were 1:1, and further stirred for 30 minutes to form a slurry containing calcium phosphate salt precipitate and 3-hydroxypropionic acid.

Filtration was performed using a filtration flask and a vacuum pump in order to separate the calcium phosphate salt precipitate. Then, a filtrate(A) before washing was obtained in a filtering flask, and then the filtered calcium phosphate salt precipitate was washed with 30 ml of distilled water, and then filtered to obtain a filtrate(B) after washing. Then, the calcium phosphate salt precipitate was dried in an oven at a temperature of 80°C for 20 hours to finally obtain the dried calcium phosphate salt precipitate.

A cation exchange resin tower (JCL-GF-F110, JUNG Glass Pyrex) was filled with 12 BV of cation exchange resin, and then washed with water to prepare a cation exchange resin column. At this time, TRILITE®CMP28LH (Samyang Corporation) was used as a cation exchange resin.

The entire filtrate (B) obtained above was charged into a cation exchange resin column at a space velocity (SV) of

2.4 m$^3$h-1, and then distilled water was charged into the cation exchange resin column at a space velocity (SV) of 7.2 m$^3$h-1 or less to obtain an aqueous 3-hydroxypropionic acid solution with a pH of 2.5 or less.

**Comparative Example 1**

5M sulfuric acid solution was added to the 3-hydroxypropionic acid fermentation liquid (concentration of about 100 g/L), which had undergone primary purification in Preparation Example 2, at a uniform rate for 5 minutes, so that the equivalents of 3-hydroxypropionic acid and sulfuric acid were 1:1, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. Then, a filtrate(A) before washing was obtained in a filtering flask, and then the filtered $CaSO_4$ precipitate was washed with 30 ml of distilled water, and then filtered to obtain a filtrate(B) after washing, which was concentrated (about 30 wt.%). The concentrated filtrate was passed through a cation exchange resin (TRILITE®CMP28LH, Samyang Corporation) to remove residual ionic impurities, thereby recovering a 3-hydroxypropionic acid-containing solution having a concentration of 300 g/L.

Then, the $CaSO_4$ precipitate was dried in an oven at a temperature of 40°C for 20 hours to finally obtain the dried $CaSO_4$ precipitate.

**<Test Example>**

**1. Evaluation of removal rate of calcium (Ca) element**

When converting the 3-hydroxypropionic acid salt crystal (Ca(3HP)$_2$ crystal) in Examples 1 to 5, 9, and 10 and Comparative Example 1 to 3-hydroxypropionic acid, $CaSO_4$ precipitates (or calcium phosphate salt precipitates) were produced, and thus, the content of the calcium(Ca) element removed was measured by an ion chromatography analysis.

Specifically, the content ($X_{ref}$) of calcium(Ca) element contained in the 32.0 wt.% Ca (3HP)$_2$ aqueous solution, and the content ($X_{filtration}$) of calcium(Ca) element contained in the filtrate (B) after washing were respectively analyzed, and the removal rate of the calcium (Ca) element was calculated according to the following general formula 1, and the results are shown in Table 1 below.

[General Formula 1]

$$\text{Removal rate of calcium(Ca) element (\%)} = (X_{ref} - X_{filtration})/X_{ref} * 100$$

**2. Measurement of recovery rate of 3-hydroxypropionic acid**

The recovery rate of 3-hydroxypropionic acid obtained in Examples 1 to 5, 9, and 10 and Comparative Example 1 was measured and calculated using a high-performance liquid chromatography (HPLC).

Specifically, the absolute amount(X) of 3-hydroxypropionic acid in the aqueous solution containing Ca(3HP)$_2$ crystal and the absolute amount(Y) of 3-hydroxypropionic acid in the filtrate were measured, and the recovery rate of 3-hydroxypropionic acid was calculated according to the following general formula 2.

[General Formula 2]

$$\text{Recovery rate of 3-hydroxypropionic acid (\%)} = (Y / X) * 100$$

**3. Measurement of impurity content**

Impurities contained in the solutions finally recovered in Examples 1 to 5, 9, 10 and Comparative Example 1 were measured by a nuclear magnetic resonance spectroscopy (NMR) and a high performance liquid chromatography (HPLC). On the other hand, if the impurity content was not measured, it was marked as '-'.

(1) High-Performance Liquid Chromatography (HPLC)

The ratio of impurities (glycerol, acetate and 1,3-propanediol) and 3-hydroxypropionic acid (3HP) was measured using HPLC.

For HPLC, 0.5 mM sulfuric acid ($H_2SO_4$) was flowed at a flow rate of 0.4 mL/min using an Aminex HPX-87H ion exclusion column (7.8 * 300 mm, 9 $\mu$m), the temperature of the column was 35°C, the injection volume was 5 $\mu$L, and the UV detector was set to 210 nm.

(2) $^1$H-NMR

The content ratio of impurities (glycerol, acetate, and 1,3-propanediol) and 3-hydroxypropionic acid (3HP) was analyzed using $^1$H-NMR.

100 $\mu$L of unpurified alkali metal or alkali earth metal 3-hydroxypropionic acid salt and purified 3-hydroxypropionic acid were sampled, and dissolved in 500 $\mu$L of D2O which is NMR solvent, and a solution added with 11.5 $\mu$L of dimethylformamide (DMF) where moisture was removed was transferred to an NMR tube to prepare a sample for $^1$H-NMR measurement.

$^1$H-NMR spectrum (AVANCE III HD FT-NMR spectrometer (500 MHz for 1H), manufactured by Bruker) was measured using the sample for measurement.

[Table 1]

|  | Removal rate of calcium(Ca) element (%) | Recovery rate of 3-hydroxypropionic acid (%) | Impurity content (ppm) | | |
|---|---|---|---|---|---|
|  |  |  | Glycerol | Acetate | 1,3-propanol |
| Example 1 | 99.0 | 69.68 | - | - | - |
| Example 2 | 98.0 | 99.50 | - | - | - |
| Example 3 | 99.0 | 99.10 | - | - | - |
| Example 4 | 99.0 | 99.80 | Detection limit | Detection limit | Detection limit |
| Example 5 | 99.9 | 95.20 | Detection limit | Detection limit | Detection limit |
| Example 9 | 90.0 | 92.00 | Detection limit | 110 | Detection limit |
| Example 10 | 90.0 | 91.00 | Detection limit | 120 | Detection limit |
| Comparative Example 1 | 95.0 | 90.00 | 2451 | 455 | 120 |

Referring to Table 1, it was confirmed that in Examples 1 to 5, 9 and 10, 3-hydroxypropionic acid was recovered by about 69.68% or more, while calcium element was removed by 90% or more. It was also confirmed that in Examples 4 and 5 in which 3-hydroxypropionic acid was purified through electrodialysis, no impurities were detected.

Thereby, it could be confirmed that according to the recovery process of 3-hydroxypropionic acid of Examples, the cation can be effectively separated from 3-hydroxypropionic acid salt to efficiently produce high-purity 3-hydroxypropionic acid.

**4. Analysis of precipitate components**

Conversion of 3-hydroxypropionic acid salt crystal ($Ca(3HP)_2$ crystals) to 3-hydroxypropionic acid appears to form a precipitate containing $CaSO_4$, and specific component analysis for such precipitates was performed using an X-ray fluorescence spectrometer (XRF).

Specifically, primary element chemical analysis was performed on a ThermoARL Advant'X Sequential XRF with Uniquant standardless software and loss on ignition (LOI) normalization (moisture content included in the normalization), the loss on ignition (LOI) value was measured in an electric furnace maintained at 975°C, and the crystal moisture was determined by the LOI difference in a drying oven at 250°C.

[Table 2]

| Component (wt.%) | $SiO_2$ | $Al_2O_3$ | $Fe_2O_3$ | CaO | MgO | $SO_3$ | LOI | Crystal moisture |
|---|---|---|---|---|---|---|---|---|
| Example 1 | 0.53 | 0.68 | 0.42 | 31.19 | - | 52.83 | 13.44 | 12.38 |
| Example 2 | - | 0.50 | 0.35 | 30.12 | - | 49.13 | 18.77 | 17.8 |

Then, the type and crystal form of the material were measured for the obtained precipitate using an X-ray diffraction analyzer (Bruker AXS D4-Endeavor XRD). Specifically, measurement was performed under the following conditions; the applied voltage was 40 kV, the applied current was 40 mA, the range of 2theta measured was 10° to 90°, and it was scanned at intervals of 0.05°.

[Table 3]

| wt.% | Gypsums | | | | Minerals | | | | | |
|---|---|---|---|---|---|---|---|---|---|---|
| | Gypsum dihydrate | Gypsum hemihydrate | Gypsum anhydrite | subtotal | calcite | dolomite | illite | chlorite | microcline | subtotal |
| Example 1 | 1.56 | 42.57 | 51.69 | 95.82 | 0.16 | 0.78 | 0.41 | 0.27 | 2.56 | 4.18 |
| Example 2 | 72.04 | 23.65 | 2.12 | 97.81 | - | 0.22 | 1.04 | - | 0.83 | 2.09 |

Referring to Tables 2 and 3, it was confirmed that the precipitate obtained in Exampless contains 95.82% or more of gypsum dihydrate, gypsum hemihydrate and gypsum anhydrite based on the total content, contains almost no minerals such as calcite and dolomite, and also contains almost no impurities such as $SiO_2$.

## 5. Analysis of the crystal phase of the precipitate

It was confirmed that in Examples 6 to 8, the type and crystal form of the materials were measured for solids A and B using an X-ray diffraction analyzer (Bruker AXS D4-Endeavor XRD). Specifically, measurement was performed under the following conditions; the applied voltage was 40 kV, the applied current was 40 mA, and Cu-K$\alpha$ radiation (wavelength $\lambda$ = 1.54184 Å) was irradiated, and the range of 2theta measured was 10° to 90°, and it was scanned at intervals of 0.05°.

The diffraction angle ($2\theta \pm 0.2°$) at which the main peak appears in the results of XRD analysis of the solids A and B of Example 6, and the diffraction angle ($2\theta \pm 0.2°$) at which the main peak appears in the results of standard XRD analysis of calcium hydroxide ($Ca(OH)_2$) are shown in Table 4 below.

The diffraction angle ($2\theta \pm 0.2°$) at which the main peak appears in the results of XRD analysis of solids A and B of Example 7, and the diffraction angle ($2\theta \pm 0.2°$) at which the main peak appears in the results of standard XRD analysis of calcium hydroxide ($Ca(OH)_2$) are shown in Table 5 below.

The diffraction angle ($2\theta \pm 0.2°$) at which the main peak appears in the results of XRD analysis of solids A and B of Example 8, and the diffraction angle ($2\theta \pm 0.2°$) at which the main peak appears in the results of standard XRD analysis of calcium carbonate are shown in Table 6 below.

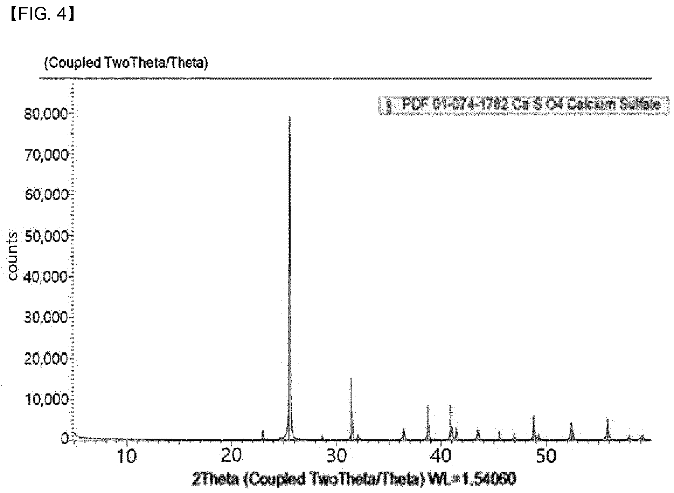

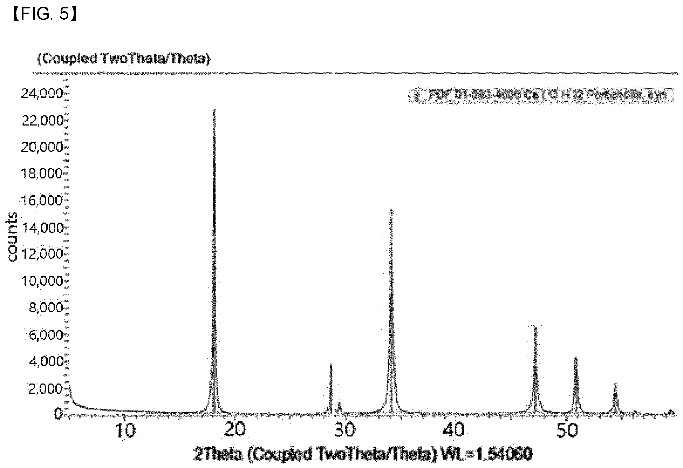

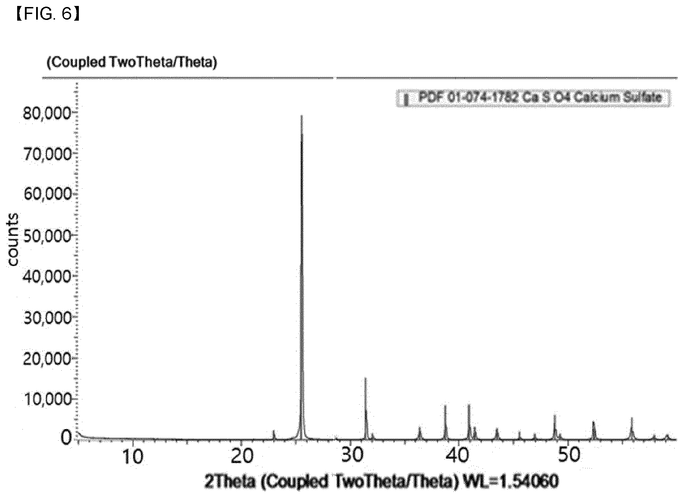

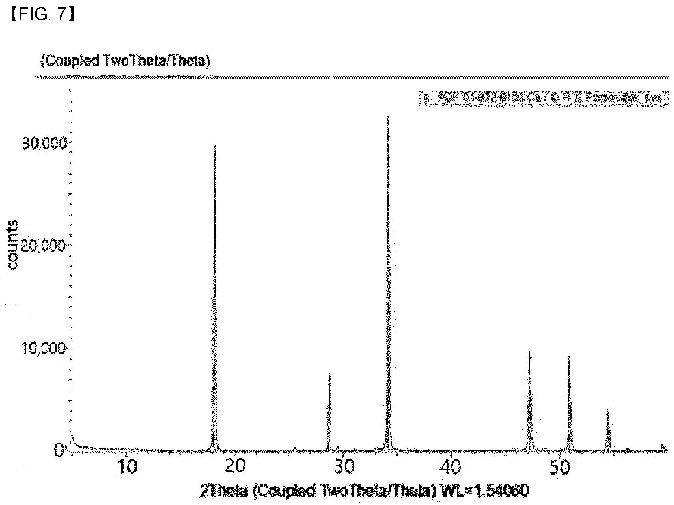

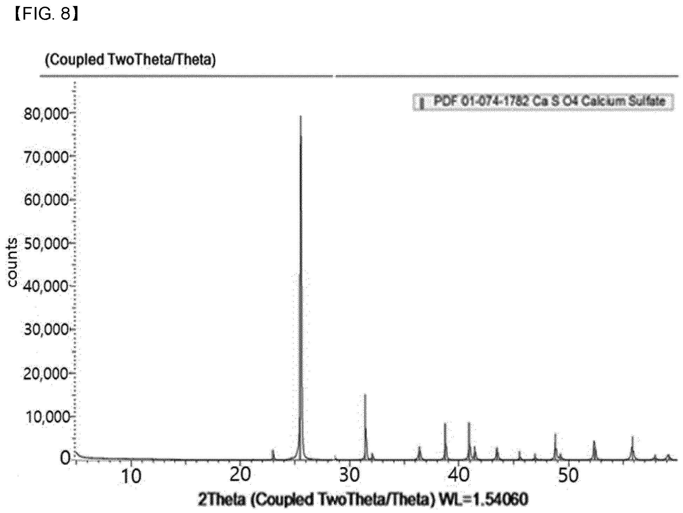

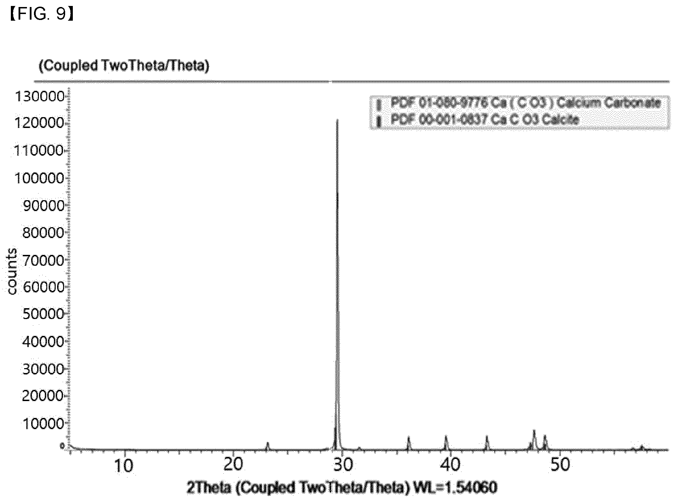

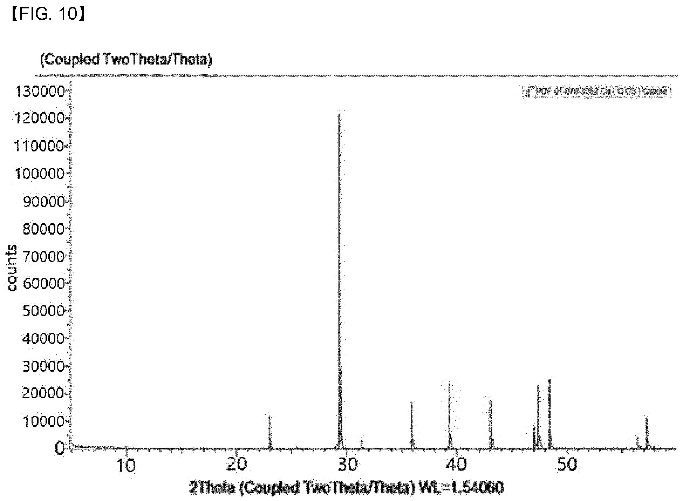

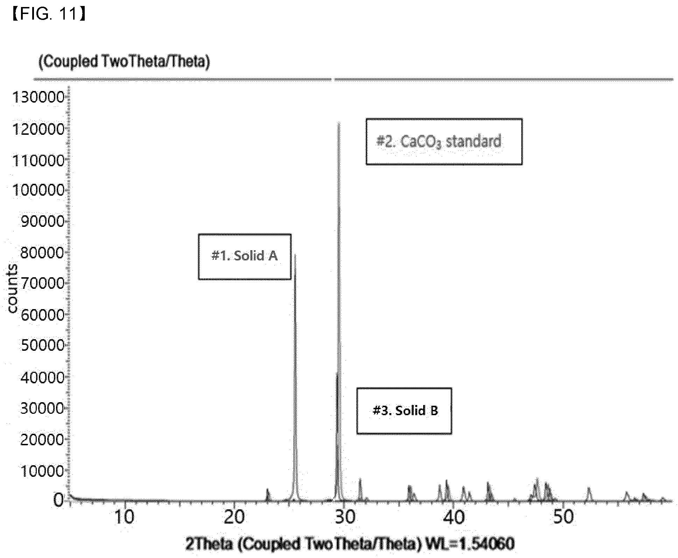

On the other hand, FIG. 4 shows the results of X-ray diffraction analysis of solid A ($CaSO_4$) of Example 6, and FIG. 5 shows the results of standard X-ray diffraction analysis of calcium hydroxide ($Ca(OH)_2$). Further, FIG. 6 shows the results of X-ray diffraction analysis of solid A ($CaSO_4$) of Example 7, and FIG. 7 shows the results of X-ray diffraction analysis of solid B of Example 7. Further, FIG. 8 shows the results of X-ray diffraction analysis of solid A ($CaSO_4$) of Example 8, FIG. 9 shows the results of standard X-ray diffraction analysis of calcium carbonate ($CaCO_3$), FIG. 10 shows the results of X-ray diffraction analysis of solid B of Example 8, and FIG. 11 is a graph overlapping X-ray diffraction analysis results of solid A($CaSO_4$), calcium carbonate and solid B of Example 8.

[Table 4]

|  | Diffraction angle ($2\theta \pm 0.2°$) at which the main peak appears |
| --- | --- |
| Solid A ($CaSO_4$) | 23, 25.4, 28.6, 31.4, 32, 36.4, 38.8, 40.9, 41.4, 43.4, 45.6, 46.9, 48.8, 49.2, 52.4, 55.8, 57.9, 59.2 |
| Standard calcium hydroxide | 18, 28.8, 34, 47.2, 51, 54.4, 56.2, 59.5 |
| Solid B | 18, 28.8, 34, 47.2, 51, 54.4, 56.2, 59.5 |

[Table 5]

|  | Diffraction angle ($2\theta \pm 0.2°$) at which the main peak appears |
| --- | --- |
| Solid A ($CaSO_4$) | 23, 25.4, 28.6, 31.4, 32, 36.4, 38.8, 40.9, 41.4, 43.4, 45.6, 46.9, 48.8, 49.2, 52.4, 55.8, 57.9, 59.2 |
| Standard calcium hydroxide | 18, 28.8, 34, 47.2, 51, 54.4, 56.2, 59.5 |
| Solid B | 18, 28.8, 34, 47.2, 51, 54.4, 56.2, 59.5 |

Referring to Tables 4 and 5, it was confirmed that the XRD pattern of the solid B of Examples 6 and 7 was identical the standard XRD pattern of calcium hydroxide, and especially, the XRD pattern of the solid A were not observed in the XRD pattern of the solid B, and thus, the reaction between gypsum anhydrite ($CaSO_4$) and ammonia water (or sodium hydroxide) was carried out at a conversion rate of 100%, so that no gypsum anhydrite ($CaSO_4$) remained, and calcium hydroxide was produced.

[Table 6]

|  | Diffraction angle ($2\theta \pm 0.2°$) at which the main peak appears |
| --- | --- |
| Solid A ($CaSO_4$) | 23, 25.4, 28.6, 31.4, 32, 36.4, 38.8, 40.9, 41.4, 43.4, 45.6, 46.9, 48.8, 49.2, 52.4, 55.8, 57.9, 59.2 |
| Standard calcium carbonate | 23, 29.2, 31.2, 36, 39.4, 43.2, 47.4, 48.5, 57.5 |

(continued)

| | Diffraction angle (2θ ± 0.2 °) at which the main peak appears |
|---|---|
| Solid B | 23, 29.2, 31.2, 36, 39.4, 43, 47, 47.4, 48.5, 56.4, 57.2 |

Referring to Table 6, it was confirmed that the XRD pattern of the solid B of Example 8 was identical to the standard XRD pattern of calcium carbonate, especially, the XRD pattern of the solid A was not observed in the XRD pattern of the solid B, and the reaction between gypsum anhydrite ($CaSO_4$) and ammonium carbonate (($NH_4$)$_2CO_3$) was carried out at a conversion rate of 100%, so that no gypsum anhydrite ($CaSO_4$) remained, and calcium carbonate was produced.

**2. Recovery rate of alkali metal hydroxide** or alkali earth metal hydroxideThe recovery rates of the alkali metal hydroxides or alkali earth metal hydroxides (solid B) of Examples 6 and 7 and the metal carbonates (solid B) of Example 8 were measured by a high performance liquid chromatography (HPLC), and the results are shown in Table 7 below. Specifically, the content (Y) of the alkali metal salt or alkali earth metal salt contained in the concentrate and the content (X) of the hydroxide/carbonate (solid B) of the finally recovered alkali metal or alkali earth metal was measured, which were respectively substituted into the following general formula 3 to calculate the recovery rate of the alkali metal hydroxide or alkali earth metal hydroxide.

[General Formula 3]

Recovery rate of alkali metal hydroxide or alkali earth metal hydroxide /carbonate (%) = X/Y * 100

[Table 7]

| | Recovery rate of alkali metal hydroxide or alkali earth metal hydroxide /carbonate (%) |
|---|---|
| Example 6 | 85 |
| Example 7 | 80 |
| Example 8 | 89 |

According to Table 7, it was confirmed that 80% or more of the alkali metal salt or alkali earth metal used in the process of fermenting 3-hydroxypropionic acid or forming 3-hydroxypropionic acid salt crystal was finally recovered in the form of alkali metal hydroxide or alkali earth metalhydroxide (or alkali metal carbonate or alkali earth metal carbonate).

## Claims

1.  A process of recovering an alkali metal salt hydrate or an alkali earth metal salt hydrate; and 3-hydroxypropionic acid, comprising:

    forming and separating a 3-hydroxypropionic acid salt crystal from a concentrate containing 3-hydroxypropionic acid in the presence of an alkali metal salt or an alkali earth metal salt;
    forming an aqueous solution containing the 3-hydroxypropionic acid salt crystal; and
    adding an acid to the aqueous solution containing the 3-hydroxypropionic acid salt crystal to form and separate the alkali metal salt hydrate or the alkali earth metal salt hydrate; and the 3-hydroxypropionic acid.

2.  The process of recovering an alkali metal salt hydrate or an alkali earth metal salt hydrate; and 3-hydroxypropionic acid according to claim 1, wherein:
    the aqueous solution containing 3-hydroxypropionic acid salt crystal contains 100 g/L or more of the 3-hydroxypropionic acid salt crystal.

3.  The process of recovering an alkali metal salt hydrate or an alkali earth metal salt hydrate; and 3-hydroxypropionic acid according to claim 1, wherein:

the adding an acid to the aqueous solution containing the 3-hydroxypropionic acid salt crystal to form and separate the alkali metal salt hydrate or the alkali earth metal salt hydrate; and the 3-hydroxypropionic acid comprises,

separating the alkali metal salt hydrate or the alkali earth metal salt hydrate from the acid-added solution containing the alkali metal salt hydrate or the alkali earth metal salt hydrate; and the 3-hydroxypropionic acid; and electrodialyzing the acid-added solution from which the alkali metal salt hydrate or the alkali earth metal salt hydrate has been separated.

4.  The process of recovering an alkali metal salt hydrate or an alkali earth metal salt hydrate; and 3-hydroxypropionic acid according to claim 3, wherein:
the electrodialyzing the acid-added solution from which the alkali metal salt hydrate or the alkali earth metal salt hydrate has been separated is carried out until the time point when the electrical conductivity in a desalination tank for recovering a desalting product formed by electrodialysis of the acid-added solution from which an alkali metal salt hydrate or an alkali earth metal salt hydrate has been separated is reduced to 50% or less of the initial value.

5.  The process of recovering an alkali metal salt hydrate or an alkali earth metal salt hydrate; and 3-hydroxypropionic acid according to claim 1, further comprising:
reacting the alkali metal salt hydrate or the alkali earth metal salt hydrate with ammonia water to form an alkali metal hydroxide or an alkali earth metal hydroxide.

6.  The process of recovering an alkali metal salt hydrate or an alkali earth metal salt hydrate; and 3-hydroxypropionic acid according to claim 1, further comprising:
adding the alkali metal salt hydrate or the alkali earth metal salt hydrate to a hydroxide ion ($OH^-$)-containing solution to form an alkali metal hydroxide or an alkali earth metal hydroxide.

7.  The process of recovering an alkali metal salt hydrate or an alkali earth metal salt hydrate; and 3-hydroxypropionic acid according to claim 5, wherein:
the alkali metal hydroxide or the alkali earth metal hydroxide is reused as the alkali metal salt or the alkali earth metal salt in the forming and separating a 3-hydroxypropionic acid salt crystal from the concentrate containing 3-hydroxypropionic acid.

8.  The process of recovering an alkali metal salt hydrate or an alkali earth metal salt hydrate; and 3-hydroxypropionic acid according to claim 5, further comprising:

fermenting a bacterium strain having 3-hydroxypropionic acid-producing ability to produce a 3-hydroxypropionic acid fermentation liquid; and
concentrating the fermentation liquid to form the concentrate containing 3-hydroxypropionic acid,
wherein the alkali metal hydroxide or the alkali earth metal hydroxide is reused during the fermentation to thereby carry out neutral fermentation.

9.  The process of recovering an alkali metal salt hydrate or an alkali earth metal salt hydrate; and 3-hydroxypropionic acid according to claim 1, further comprising:
reacting the alkali metal salt hydrate or the alkali earth metal salt hydrate with a carbonate to form an alkali metal carbonate or an alkali earth metal carbonate.

10. The process of recovering an alkali metal salt hydrate or an alkali earth metal salt hydrate; and 3-hydroxypropionic acid according to claim 1, wherein:

the adding an acid to the aqueous solution containing the 3-hydroxypropionic acid salt crystal to form and separate the alkali metal salt hydrate or the alkali earth metal salt hydrate; and 3-hydroxypropionic acid comprises,
contacting the formed solution containing 3-hydroxypropionic acid with a cation exchange resin column; and
recovering the 3-hydroxypropionic acid from the solution contacted with the cation exchange resin column.

11. The process of recovering an alkali metal salt hydrate or an alkali earth metal salt hydrate; and 3-hydroxypropionic acid according to claim 1, wherein:
at the time point when an acid is added to an aqueous solution containing the 3-hydroxypropionic acid salt crystal, the temperature of the aqueous solution containing the 3-hydroxypropionic acid salt crystal is 0°C or more and 90°C or less.

12. The process of recovering an alkali metal salt hydrate or an alkali earth metal salt hydrate; and 3-hydroxypropionic acid according to claim 1, wherein:
when an acid is added to an aqueous solution containing the 3-hydroxypropionic acid salt crystal, a precipitate having a particle size of 0.5 $\mu$m or more and 500.0 $\mu$m or less is formed.

13. The process of recovering an alkali metal salt hydrate or an alkali earth metal salt hydrate; and 3-hydroxypropionic acid according to claim 12, wherein:
a moisture content of the precipitate is 200% or less.

14. The process of recovering an alkali metal salt hydrate or an alkali earth metal salt hydrate; and 3-hydroxypropionic acid according to claim 1, wherein:
the concentrate contains 300 g/L or more of the 3-hydroxypropionic acid.

15. The process of recovering an alkali metal salt hydrate or an alkali earth metal salt hydrate; and 3-hydroxypropionic acid according to claim 1, wherein:
the alkali earth metal salt is $Ca(OH)_2$, $Mg(OH)_2$ or a mixture thereof.

**Patentansprüche**

1. Verfahren zur Gewinnung eines Alkalimetallsalzhydrats oder eines Erdalkalimetallsalzhydrats; und 3-Hydroxypropionsäure, umfassend:

Bilden und Abtrennen eines 3-Hydroxypropionsäuresalzkristalls aus einem Konzentrat, das 3-Hydroxypropionsäure enthält, in Gegenwart eines Alkalimetallsalzes oder eines Erdalkalimetallsalzes;
Bilden einer wässrigen Lösung, die den 3-Hydroxypropionsäuresalzkristall enthält; und
Zugeben einer Säure zu der wässrigen Lösung, die den 3-Hydroxypropionsäuresalzkristall enthält, um das Alkalimetallsalzhydrat oder das Erdalkalimetallsalzhydrat zu bilden und abzutrennen; und die 3-Hydroxypropionsäure.

2. Verfahren zur Gewinnung eines Alkalimetallsalzhydrats oder eines Erdalkalimetallsalzhydrats; und 3-Hydroxypropionsäure nach Anspruch 1, wobei:
die wässrige Lösung, die den 3-Hydroxypropionsäuresalzkristall enthält, 100 g/l oder mehr des 3-Hydroxypropionsäuresalzkristalls enthält.

3. Verfahren zur Gewinnung eines Alkalimetallsalzhydrats oder eines Erdalkalimetallsalzhydrats; und 3-Hydroxypropionsäure nach Anspruch 1, wobei:

das Zugeben einer Säure zu der wässrigen Lösung, die den 3-Hydroxypropionsäuresalzkristall enthält, um das Alkalimetallsalzhydrat oder das Erdalkalimetallsalzhydrat zu bilden und abzutrennen; und die 3-Hydroxypropionsäure umfasst,
Abtrennen des Alkalimetallsalzhydrats oder des Erdalkalimetallsalzhydrats aus der mit Säure versetzten Lösung, die das Alkalimetallsalzhydrat oder das Erdalkalimetallsalzhydrat enthält; und die 3-Hydroxypropionsäure; und
Elektrodialysieren der mit Säure versetzten Lösung, aus der das Alkalimetallsalzhydrat oder das Erdalkalimetallsalzhydrat abgetrennt wurde.

4. Verfahren zur Gewinnung eines Alkalimetallsalzhydrats oder eines Erdalkalimetallsalzhydrats; und 3-Hydroxypropionsäure nach Anspruch 3, wobei:
das Elektrodialysieren der mit Säure versetzten Lösung, aus der das Alkalimetallsalzhydrat oder das Erdalkalimetallsalzhydrat abgetrennt wurde, bis zu dem Zeitpunkt durchgeführt wird, an dem die elektrische Leitfähigkeit in einem Entsalzungstank zur Gewinnung eines Entsalzungsprodukts, das durch Elektrodialyse der mit Säure versetzten Lösung gebildet wurde, aus der ein Alkalimetallsalzhydrat oder ein Erdalkalimetallsalzhydrat abgetrennt wurde, auf 50 % oder weniger des Ausgangswerts reduziert wird.

5. Verfahren zur Gewinnung eines Alkalimetallsalzhydrats oder eines Erdalkalimetallsalzhydrats; und 3-Hydroxy-

27

propionsäure nach Anspruch 1, ferner umfassend:
Umsetzen des Alkalimetallsalzhydrats oder des Erdalkalimetallsalzhydrats mit Ammoniakwasser, um ein Alkalimetallhydroxid oder ein Erdalkalimetallhydroxid zu bilden.

6. Verfahren zur Gewinnung eines Alkalimetallsalzhydrats oder eines Erdalkalimetallsalzhydrats; und 3-Hydroxypropionsäure nach Anspruch 1, ferner umfassend:
Zugeben des Alkalimetallsalzhydrats oder des Erdalkalimetallsalzhydrats zu einer Hydroxidionen (OH⁻) enthaltenden Lösung, um ein Alkalimetallhydroxid oder ein Erdalkalimetallhydroxid zu bilden.

7. Verfahren zur Gewinnung eines Alkalimetallsalzhydrats oder eines Erdalkalimetallsalzhydrats; und 3-Hydroxypropionsäure nach Anspruch 5, wobei:
das Alkalimetallhydroxid oder das Erdalkalimetallhydroxid als das Alkalimetallsalz oder das Erdalkalimetallsalz beim Bilden und Abtrennen eines 3-Hydroxypropionsäuresalzkristalls aus dem Konzentrat, das 3-Hydroxypropionsäure enthält, wiederverwendet wird.

8. Verfahren zur Gewinnung eines Alkalimetallsalzhydrats oder eines Erdalkalimetallsalzhydrats; und 3-Hydroxypropionsäure nach Anspruch 5, ferner umfassend:

Fermentieren eines Bakterienstamms mit 3-Hydroxypropionsäure-Produktionsfähigkeit, um eine 3-Hydroxypropionsäure-Fermentationsflüssigkeit zu produzieren; und
Konzentrieren der Fermentationsflüssigkeit, um das Konzentrat zu bilden, das 3-Hydroxypropionsäure enthält, wobei das Alkalimetallhydroxid oder das Erdalkalimetallhydroxid während der Fermentation wiederverwendet wird, um dadurch eine neutrale Fermentation durchzuführen.

9. Verfahren zur Gewinnung eines Alkalimetallsalzhydrats oder eines Erdalkalimetallsalzhydrats; und 3-Hydroxypropionsäure nach Anspruch 1, ferner umfassend:
Umsetzen des Alkalimetallsalzhydrats oder des Erdalkalimetallsalzhydrats mit einem Carbonat, um ein Alkalimetallcarbonat oder ein Erdalkalimetallcarbonat zu bilden.

10. Verfahren zur Gewinnung eines Alkalimetallsalzhydrats oder eines Erdalkalimetallsalzhydrats; und 3-Hydroxypropionsäure nach Anspruch 1, wobei:

das Zugeben einer Säure zu der wässrigen Lösung, die den 3-Hydroxypropionsäuresalzkristall enthält, um das Alkalimetallsalzhydrat oder das Erdalkalimetallsalzhydrat zu bilden und abzutrennen; und die 3-Hydroxypropionsäure umfasst,
Inkontaktbringen der gebildeten Lösung, die 3-Hydroxypropionsäure enthält, mit einer Kationenaustauschharzsäule; und
Gewinnen der 3-Hydroxypropionsäure aus der Lösung, die mit der Kationenaustauschharzsäule in Kontakt gebracht wurde.

11. Verfahren zur Gewinnung eines Alkalimetallsalzhydrats oder eines Erdalkalimetallsalzhydrats; und 3-Hydroxypropionsäure nach Anspruch 1, wobei:
zu dem Zeitpunkt, an dem eine Säure zu einer wässrigen Lösung zugegeben wird, die den 3-Hydroxypropionsäuresalzkristall enthält, die Temperatur der wässrigen Lösung, die den 3-Hydroxypropionsäuresalzkristall enthält, 0 °C oder mehr und 90 °C oder weniger beträgt.

12. Verfahren zur Gewinnung eines Alkalimetallsalzhydrats oder eines Erdalkalimetallsalzhydrats; und 3-Hydroxypropionsäure nach Anspruch 1, wobei:
wenn eine Säure zu einer wässrigen Lösung zugegeben wird, die den 3-Hydroxypropionsäuresalzkristall enthält, ein Niederschlag mit einer Teilchengröße von 0,5 μm oder mehr und 500,0 μm oder weniger gebildet wird.

13. Verfahren zur Gewinnung eines Alkalimetallsalzhydrats oder eines Erdalkalimetallsalzhydrats; und 3-Hydroxypropionsäure nach Anspruch 12, wobei:
ein Feuchtigkeitsgehalt des Niederschlags 200% oder weniger beträgt.

14. Verfahren zur Gewinnung eines Alkalimetallsalzhydrats oder eines Erdalkalimetallsalzhydrats; und 3-Hydroxypropionsäure nach Anspruch 1, wobei:
das Konzentrat 300 g/l oder mehr der 3-Hydroxypropionsäure enthält.

**15.** Verfahren zur Gewinnung eines Alkalimetallsalzhydrats oder eines Erdalkalimetallsalzhydrats; und 3-Hydroxypropionsäure nach Anspruch 1, wobei:
das Erdalkalimetallsalz Ca(OH)$_2$ , Mg(OH)$_2$ oder eine Mischung davon ist.

**Revendications**

**1.** Processus de récupération d'un hydrate de sel de métal alcalin ou d'un hydrate de sel alcalino-terreux ; et d'acide 3-hydroxypropionique, consistant à :

former et séparer un cristal de sel d'acide 3-hydroxypropionique d'un concentré contenant de l'acide 3-hydroxypropionique en présence d'un sel de métal alcalin ou d'un sel de métal alcalino-terreux ;
former une solution aqueuse contenant le cristal de sel d'acide 3-hydroxypropionique ; et
ajouter un acide à la solution aqueuse contenant le cristal de sel d'acide 3-hydroxypropionique pour former et séparer l'hydrate de sel de métal alcalin ou l'hydrate de sel alcalino-terreux ; et l'acide 3-hydroxypropionique.

**2.** Processus de récupération d'un hydrate de sel de métal alcalin ou d'un hydrate de sel alcalino-terreux ; et d'acide 3-hydroxypropionique selon la revendication 1, dans lequel :
la solution aqueuse contenant un cristal de sel d'acide 3-hydroxypropionique contient 100 g/L ou plus du cristal de sel d'acide 3-hydroxypropionique.

**3.** Processus de récupération d'un hydrate de sel de métal alcalin ou d'un hydrate de sel alcalino-terreux ; et d'acide 3-hydroxypropionique selon la revendication 1, dans lequel :

l'ajout d'un acide à la solution aqueuse contenant le cristal de sel d'acide 3-hydroxypropionique pour former et séparer l'hydrate de sel de métal alcalin ou l'hydrate de sel alcalino-terreux ; et l'acide 3-hydroxypropionique consiste à,
séparer l'hydrate de sel de métal alcalin ou l'hydrate de sel alcalino-terreux de la solution avec acide ajouté contenant l'hydrate de sel de métal alcalin ou l'hydrate de sel alcalino-terreux ; et l'acide 3-hydroxypropionique ; et électrodialyser la solution avec acide ajouté dont l'hydrate de sel de métal alcalin ou l'hydrate de sel alcalino-terreux a été séparé.

**4.** Processus de récupération d'un hydrate de sel de métal alcalin ou d'un hydrate de sel alcalino-terreux ; et d'acide 3-hydroxypropionique selon la revendication 3, dans lequel :
l'électrodialyse de la solution avec acide ajouté dont l'hydrate de sel de métal alcalin ou l'hydrate de sel alcalino-terreux a été séparé est effectuée jusqu'au moment où la conductivité électrique dans un réservoir de dessalement pour récupérer un produit de dessalement formé par l'électrodialyse de la solution avec acide ajouté dont un hydrate de sel de métal alcalin ou un hydrate de sel alcalino-terreux a été séparé est réduite à 50 % ou moins de la valeur initiale.

**5.** Processus de récupération d'un hydrate de sel de métal alcalin ou d'un hydrate de sel alcalino-terreux ; et d'acide 3-hydroxypropionique selon la revendication 1, consistant en outre à :
faire réagir l'hydrate de sel de métal alcalin ou l'hydrate de sel alcalino-terreux avec de l'eau ammoniacale pour former un hydroxyde de métal alcalin ou un hydroxyde de métal alcalino-terreux.

**6.** Processus de récupération d'un hydrate de sel de métal alcalin ou d'un hydrate de sel alcalino-terreux ; et d'acide 3-hydroxypropionique selon la revendication 1, consistant en outre à :
ajouter l'hydrate de sel de métal alcalin ou l'hydrate de sel alcalino-terreux à une solution contenant un ion d'hydroxyde (OH⁻) pour former un hydroxyde de métal alcalin ou un hydroxyde de métal alcalino-terreux.

**7.** Processus de récupération d'un hydrate de sel de métal alcalin ou d'un hydrate de sel alcalino-terreux ; et d'acide 3-hydroxypropionique selon la revendication 5, dans lequel :
l'hydroxyde de métal alcalin ou l'hydroxyde de métal alcalino-terreux est réutilisé en tant que sel de métal alcalin ou sel de métal alcalino-terreux dans la formation et la séparation d'un cristal de sel d'acide 3-hydroxypropionique à partir du concentré contenant de l'acide 3-hydroxypropionique.

**8.** Processus de récupération d'un hydrate de sel de métal alcalin ou d'un hydrate de sel alcalino-terreux ; et d'acide 3-hydroxypropionique selon la revendication 5, consistant en outre à :

faire fermenter une souche bactérienne ayant la capacité de produire de l'acide 3-hydroxypropionique pour produire un liquide de fermentation d'acide 3-hydroxypropionique ; et

concentrer le liquide de fermentation pour former le concentré contenant de l'acide 3-hydroxypropionique, dans lequel l'hydroxyde de métal alcalin ou l'hydroxyde de métal alcalino-terreux est réutilisé pendant la fermentation pour réaliser ainsi une fermentation neutre.

9. Processus de récupération d'un hydrate de sel de métal alcalin ou d'un hydrate de sel alcalino-terreux ; et d'acide 3-hydroxypropionique selon la revendication 1, consistant en outre à :

faire réagir l'hydrate de sel de métal alcalin ou l'hydrate de sel alcalino-terreux avec un carbonate pour former un carbonate de métal alcalin ou un carbonate de métal alcalino-terreux.

10. Processus de récupération d'un hydrate de sel de métal alcalin ou d'un hydrate de sel alcalino-terreux ; et d'acide 3-hydroxypropionique selon la revendication 1, dans lequel :

l'ajout d'un acide à la solution aqueuse contenant un cristal de sel d'acide 3-hydroxypropionique pour former et séparer l'hydrate de sel de métal alcalin ou l'hydrate de sel alcalino-terreux ; et l'acide 3-hydroxypropionique consiste à,

mettre en contact la solution formée contenant l'acide 3-hydroxypropionique avec une colonne de résine échangeuse de cations ; et

récupérer l'acide 3-hydroxypropionique de la solution en contact avec la colonne de résine échangeuse de cations.

11. Processus de récupération d'un hydrate de sel de métal alcalin ou d'un hydrate de sel alcalino-terreux ; et d'acide 3-hydroxypropionique selon la revendication 1, dans lequel :

au moment où un acide est ajouté à une solution aqueuse contenant le cristal de sel d'acide 3-hydroxypropionique, la température de la solution aqueuse contenant le cristal de sel d'acide 3-hydroxypropionique est égale ou supérieure à 0°C et égale ou inférieure à 90°C.

12. Processus de récupération d'un hydrate de sel de métal alcalin ou d'un hydrate de sel alcalino-terreux ; et d'acide 3-hydroxypropionique selon la revendication 1, dans lequel :

quand un acide est ajouté à une solution aqueuse contenant le cristal de sel d'acide 3-hydroxypropionique, un précipité ayant une taille de particule égale ou supérieure à 0,5 $\mu$m et égale ou inférieure à 500,0 $\mu$m est formé.

13. Processus de récupération d'un hydrate de sel de métal alcalin ou d'un hydrate de sel alcalino-terreux ; et d'acide 3-hydroxypropionique selon la revendication 12, dans lequel :

une teneur en humidité du précipité est égale ou inférieure à 200 %.

14. Processus de récupération d'un hydrate de sel de métal alcalin ou d'un hydrate de sel alcalino-terreux ; et d'acide 3-hydroxypropionique selon la revendication 1, dans lequel :

le concentré contient 300 g/L ou plus de l'acide 3-hydroxypropionique.

15. Processus de récupération d'un hydrate de sel de métal alcalin ou d'un hydrate de sel alcalino-terreux ; et d'acide 3-hydroxypropionique selon la revendication 1, dans lequel :

le sel de métal alcalino-terreux est Ca(OH)$_2$, Mg(OH)$_2$ ou un mélange de ces derniers.

【FIG. 1】

【FIG. 2】

| | |
|---|---|
| Quartz | 0.07 % |
| Gypsum | 72.04 % |
| Bassanite | 23.65 % |
| Anhydrite | 2.12 % |
| Dolomite | 0.22 % |
| Illite | 1.04 % |
| Microcline | 0.83 % |
| Pyrite | 0.03 % |

【FIG. 3】

Concentration tank (waste liquid)

Desalination tank (purified 3HP)

【FIG. 4】

【FIG. 5】

【FIG. 6】

【FIG. 7】

【FIG. 8】

【FIG. 9】

【FIG. 10】

(Coupled TwoTheta/Theta)

【FIG. 11】

**EP 4 328 215 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20210012270

- KR 1020200051375